# EUROPEAN PATENT SPECIFICATION

(11) **EP 1 108 786 B1**
(45) Date of publication and mention of the grant of the patent: **13.12.2006**
(21) Application number: 99938607.1
(22) Date of filing: 24.08.1999
(51) Int. Cl.: C12N 15/12, A01K 67/027, C07K 14/785, C07K 16/18, G01N 33/50, C12Q 1/68

(54) **NOVEL COLLECTIN**
NEUARTIGES COLLECTIN
NOUVELLE COLLECTINE

(30) Priority: 24.08.1998 JP 23761198
(43) Date of publication of application: 20.06.2001
(73) Proprietor: FUSO PHARMACEUTICAL INDUSTRIES LTD., Osaka-shi, Osaka 541-0045 (JP)
(72) Inventor: WAKAMIYA, Nobutaka, Ibaraki-shi, Osaka 567-0826 (JP)
(74) Representative: Gardner, Rebecca Katherine
(86) International application number: PCT/JP1999/004552
(87) International publication number: WO 2000/011161

(56) References cited:
- EP-A- 0 856 580
- WO-A2-98/55614
- JP-A- 9 238 683
- US-A- 4 906 734
- LAURSEN S B ET AL: "Cloning and sequencing of a cDNA encoding chicken mannan-binding lectin (MBL) and comparison with mammalian analogues." IMMUNOLOGY, vol. 93, no. 3, March 1998 (1998-03), pages 421-430, XP000909084 ISSN: 0019-2805
- OHTANI KATSUKI ET AL: "Molecular cloning of a novel human collectin from liver (CL-L1)." JOURNAL OF BIOLOGICAL CHEMISTRY, vol. 274, no. 19, 7 May 1999 (1999-05-07), pages 13681-13689, XP002242030 ISSN: 0021-9258
- DRIEKAMER K: "TWO DISTINCT CLASSES OF CARBOHYDRATE-RECOGNITION DOMAINS IN ANIMAL LECTINS" JOURNAL OF BIOLOGICAL CHEMISTRY, AMERICAN SOCIETY OF BIOLOGICAL CHEMISTS, BALTIMORE, MD, US, vol. 263, no. 20, 15 July 1988 (1988-07-15), pages 9557-9560, XP001057596 ISSN: 0021-9258
- HOPPE H-J ET AL: "COLLECTINS - SOLUBLE PROTEINS CONTAINING COLLAGENOUS REGIONS AND LECTIN DOMAINS - AND THEIR ROLES IN INNATE IMMUNITY" PROTEIN SCIENCE, CAMBRIDGE UNIVERSITY PRESS, CAMBRIDGE, GB, vol. 3, 1994, pages 1143-1158, XP000861811 ISSN: 0961-8368
- DATABASE EMBL [Online] retrieved from EBI Database accession no. R78202 XP002242031
- ADAMS M D ET AL: "INITIAL ASSESSMENT OF HUMAN GENE DIVERSITY AND EXPRESSION PATTERNS BASED UPON 83 MILLION NUCLEOTIDES OF CDNA SEQUENCE" NATURE, MACMILLAN JOURNALS LTD. LONDON, GB, vol. 377, no. SUPPL, 28 September 1995 (1995-09-28), pages 3-174, XP002922194 ISSN: 0028-0836
- TAYLOR M E ET AL: "STRUCTURE AND EVOLUTIONARY ORIGIN OF THE GENE ENCODING A HUMAN SERUM MANNOSE-BINDING PROTEIN" BIOCHEMICAL JOURNAL, PORTLAND PRESS, LONDON, GB, vol. 262, no. 3, 1989, pages 763-771, XP000861866 ISSN: 0264-6021
- BOTAS C ET AL: "ALTERED SURFACTANT HOMEOSTASIS AND ALVEOLAR TYPE II CELL MORPHOLOGYIN MICE LACKING SURFACTANT PROTEIN D" PROCEEDINGS OF THE NATIONAL ACADEMY OF SCIENCES OF USA, NATIONAL ACADEMY OF SCIENCE. WASHINGTON, US, vol. 95, no. 20, September 1998 (1998-09), pages 11869-11874, XP001059873 ISSN: 0027-8424
- RUJNEESH MALHOTRA ET AL.: 'Interaction of Clq receptor with Lung surfactant protein A' EUR. J. IMMUNOL. vol. 22, 1992, pages 1437 - 1445, XP002926527
- YASUHIKO SUZUKI ET AL.: 'Cloning and sequencing of a cDNA coding for bovine conglutinin' BIOCHEMICAL AND BIOPHYSICAL RESEARCH COMMUNICATIONS vol. 191, no. 2, 1993, pages 335 - 342, XP002926528

## Description

### [Field of the Invention]

The present invention relates to a novel collectin which is useful for investigating mechanisms of biological defense system, and is expected to be applied for utilizing as materials for medicines because it may have physiological activities including anti-viral activities and the like.

### [Background Art]

Collectin is a generic name of proteins having Ca²⁺-dependent carbohydrate recognition region (CRD) and collagen-like region, and the member of these proteins is conceived to involve in basic immunity systems against a wide spectrum of microorganisms such as bacteria and viruses.

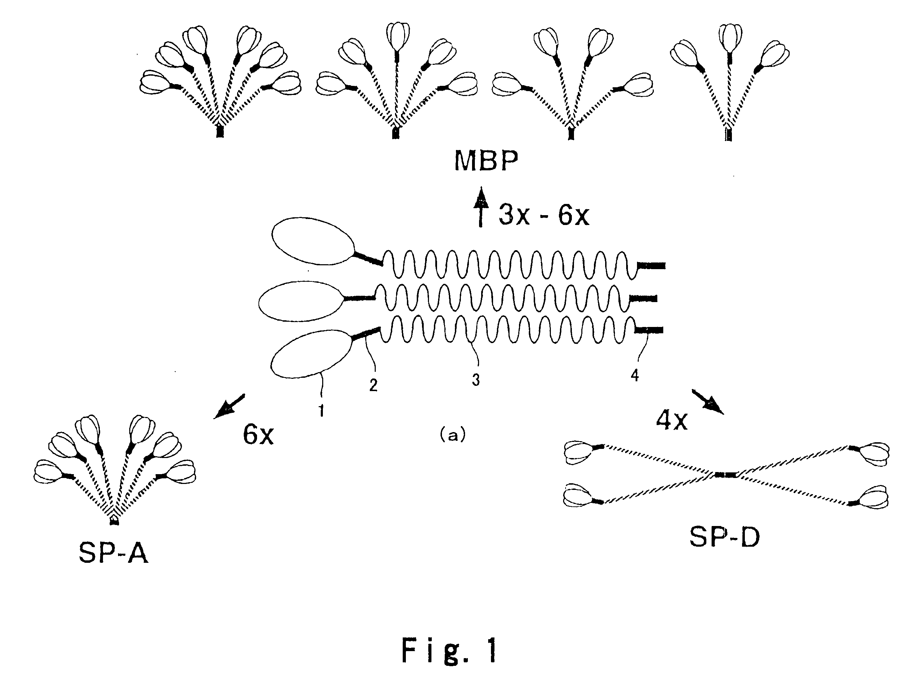

The collectins that have been identified heretofore include mannan-binding protein (MBP), surfactant protein A (SP-A), surfactant protein D (SP-D), conglutinin and the like. These collectins are known to be constituted from basic structures (Fig. 1) comprising unique regions of: (1) Ca²⁺-dependent carbohydrate recognition domain (CRD), and (2) collagen-like region [Malhortra *et al., Eur. J. Immunol.* Vol.22, 1437-1445, 1992], and a subunit may be formed from the three basic structures through making a triple helix in the collagen-like region, besides, such subunits may form an oligomer, e.g., trimer, tetramer and hexamer.

In vertebrates, mechanisms involving cellular immune responses and specific antibody reactions are considered as dominant host-defense systems against inversion of the pathogenic bacteria, viruses and the like. Recently, involvement in nonspecific immune responses of the lectins such as conglutinin has been suggested, for example, it was reported that the lectins may play important roles in neutralizing and removing the various microorganisms in infants having insufficient maternal antibodies and undeveloped specific defense systems [Super *et al., Lancet,* Vol.2, 1236-1239, 1989].

Moreover, with respect to the roles of the lectins in the biological host-defense systems, it was reported that the host becomes liable to be infected by, for example, a reduction of the MBP concentration in blood due to genetic mutation of the MBP gene [Sumiya *et al., Lancet,* Vol.337, 1569-1570, 1991].

The present inventors previously found that the conglutinin and the mannan-binding protein can inhibit infection and hemagglutination activity of H1 and H3 Type Influenza A viruses [Wakamiya et al., *Glycoconjugate J.,* Vol.8, 235, 1991; Wakamiya *et al., Biochem. Biophys. Res. Comm.,* Vol. 187, 1270-1278, 1992].

Thereafter, the present inventors isolated a cDNA clone encoding the conglutinin, and found that closer correlation may exist between the conglutinin gene and various surfactant protein D gene [Suzuki *et al., Biochem. Biophys. Res. Comm.,* Vol.191, 335-342, 1993].

Thus, the collectin has been expected to be useful in investigating mechanisms of biological defense, and useful as a physiologically active medical material. Therefore, discovery of novel molecular species belonging to this family would largely contribute to various medical fields including therapy of infectious diseases, as well as biological fields.

### [Disclosure of the Invention]

The present invention was accomplished in consideration of the aforementioned state of art, and an object of the invention is to provide a novel collectin which can be expected to exhibit physiological activities such as anti-bacterial, anti-viral activities, especially in human body.

Accordingly, the present invention provides novel collectin gene and protein having characteristic structures of the collectins, which are distinct from those reported heretofore as follows:
[1] A polynucleotide consisting of a nucleotide sequence which encodes a protein having an amino acid sequence set out in SEQ ID NO: 2, 206-547;
[2] A polynucleotide consisting of a nucleotide sequence which encodes a protein having an amino acid sequence set out in SEQ ID NO: 2, 229-547;
[3] A polynucleotide consisting of a nucleotide sequence set out in SEQ ID NO: 1, 670-1695;
[4] A polynucleotide consisting of a nucleotide sequence set out in SEQ ID NO: 1, 739-1695;
[5] A polynucleotide encoding a collectin protein, wherein said polynucleotide can hybridize under a stringent condition with a probe that is an amplification product of PCR reaction conducted using primers having nucleotide sequences set out in SEQ ID NO: 4 and SEQ ID NO: 5;
[6] A polynucleotide which can hybridize under a stringent condition with any one of the polynucleotide described in [1] to [5], wherein the protein encoded by said polynucleotide is a human collectin comprising (1) Ca²⁺-dependent carbohydrate recognition domain (CRD), and (2) collagen-like region;
[7] A novel collectin encoded by the polynucleotide of [3] to [6];
[8] A novel collectin comprising an amino acid sequence set out in SEQ ID NO:2, 206-547;
[9] A novel collectin comprising an amino acid sequence set out in SEQ ID NO:2, 229-547.

Other collectins may consist of the amino acid sequences of [7] to [9], that comprises deletion, substitution and/or addition of one or more amino acids, and wherein the amino acid sequence thereof comprises those corresponding to (1) Ca²⁺-dependent carbohydrate recognition domain (CRD), and (2) collagen-like region.

### [Brief Description of the Drawings]

Figure 1 is a schematic drawing showing basic structures and overviews of the principal collectins reported heretofore;
Figure 2 shows the alignment of the preceding half portions of amino acid sequences of three collectins reported heretofore;
Figure 3 shows the alignment of the latter half portions of the amino acid sequences in Figure 2;
Figure 4 shows each of the primers used for sequencing the novel collectin of the present invention, and the nucleotide sequences which were read out from the sequencer (b); and an ORF of the obtained novel collectin (a);
Figure 5 shows the alignment of the preceding half portions of amino acid sequences of the three collectins reported heretofore and the novel collectin of the present invention;
Figure 6 shows the alignment of the latter half portions of the amino acid sequences in Figure 5;
Figure 7 shows results of genomic Southern analysis with the novel collectin of the present invention, and the restriction enzymes employed in each of the lanes are (1) EcoRI, (2) XbaI, (3) HindIII, (4) PstI, (5) BgIII and (6) BamHI;
Figure 8 shows results of analysis of distribution of expression of mRNA in: (1) brain, (2) heart, (3) kidney, (4) spleen, (5) liver, (6) small intestine, (7) muscular tissue, (8) testis, (9) placenta, or (10) large intestine demonstrating the tissue distribution of the novel collectin of the present invention;
Figure 9 shows results of genomic Southern analysis of various vertebrates, i.e., (1) human, (2) monkey, (3) rat, (4) mouse, (5) dog, (6) cow, (7) rabbit and (8) chicken to elucidate the interspecies conservation of the novel collectin of the present invention;
Figure 10 shows a phylogenetic tree of various collectins; and
Figure I is schematic drawings illustrating a vector that expresses the novel collectin of the present invention.

### [Best Mode for Carrying Out the Invention]

In the preferred embodiment of the present invention, the above-mentioned polynucleotide in any of [1] to [6] of the present invention may be preferably cDNA.

The polynucleotide in the above [2] consisting of a nucleotide sequence encoding a protein having at least an amino acid sequence set out in SEQ ID NO: 2, 229-547, however, additional nucleotide sequence such as those encoding a protein having an amino acid sequence such as 226-228 or 211-228 of SEQ ID NO: 2, or 102-228, 91-228, 9-228, 1-228 of SEQ ID NO: 2 may be contained upstream of the first methionine residue.

Moreover, the polynucleotide set out in the above [4] may further consist of a nucleotide sequence of 730-738 or 685-738, or 358-738, 325-738, 79-738, 55-738 or 1-738 in SEQ ID NO: 1, 5' upstream thereof.

Additionally, the polynucleotide in the above [3] or [4] may consist of a nucleotide sequence of 1696-204 in SEQ ID NO:1, 3' downstream thereof.

Further, the protein [7] to [10] of the present invention mentioned above may be preferably derived from human taking into account of the usefulness as physiologically active medical materials, because it can be expected to exhibit anti-bacterial, anti-viral activities and the like in human body. Accordingly, the present invention contemplates a novel collectin derived from human, and upon examination of various human tissues, expression of a novel collectin was suggested, which was conceived to be useful.

Further, the novel collectin of the above [9] may preferably consist of at least an amino acid sequence of 229-547 in SEQ ID NO: 2, and an amino acid sequence for example, 226-228 or 211-228 in SEQ ID NO: 2, or 102-228, 91-228, 9-228, 1-228 in SEQ ID NO: 2 or the like may be additionally included upstream of the first methionine residue.

The stringent hybridization condition in the inventions of the above [5] and [6] may include for example, a series of the following steps for the hybridization: prehybridization in a solution of 5 x SSC (prepared by diluting 20 x SSC (3 M NaCl, 0.3 M sodium citrate)), 1% blocking agent (Boehringer Mannheim), 0.1% N-lauroyl sarcosine, and 0.02% SDS, at 68°C for an hour; hybridization in a solution of 5 x SSC, 1% blocking agent, 0.1% N-lauroyl sarcosine, and 0.02% SDS containing cDNA probes (10 ng/ml), at 55°C for 16 hours; washing in a solution of 2 x SSC/0.1% SDS for 5 minutes, 2 times; and washing in a solution of 0.5 x SSC/0.1% SDS at 55°C for 15 minutes, 2 times. However, several modifications/alterations of these conditions such as the concentration of the solution, incubation temperature and time may be made on the basis of the knowledge of the skilled art.
Further, deletion, substitution and/or addition of one or more amino acids in the above [10] may be carried out. Examples are those which do not result in great changes of hydrophilic/hydrophobic, or acidic/basic nature of the amino acid residues of the novel collectin, without bringing much alteration of each of the properties of (1) Ca²⁺-dependent carbohydrate recognition domain (CRD) and (2) collagen-like region. On the basis of the amino acid sequences and structures of the proteins belonging to the collectin family reported heretofore, for example, deletion, substitution and/or addition of 1-10 amino acid residues in (1) Ca²⁺-dependent carbohydrate recognition domain (CRD), and 1-100, preferably 1-15 amino acid residues in (2) collagen-like region may be allowed.

The present invention also provides a vector, which comprises the above-described polynucleotide in a manner that allows expression of the novel collectin.

The vector in which the polynucleotide is inserted may be referred to as a replicon such as plasmid, λ-phage, cosmid and the like with the above described novel collectin polynucleotide being incorporated therein so that the novel collectin can be replicated and expressed. For example, vectors capable of cloning longer DNA fragments than cosmid may include P1 phage, F factor and artificial chromosome YAC of yeast and the like. Moreover, λ-phage may include replacement vector and insertion vector, and these can be selected ad libitum taking into account of the length of gene to be introduced. Moreover, known vectors capable of being expressed in animal cells include for example, SV40 vectors, bovine papiloma virus vector, herpes virus vector, adenovirus vector, poxvirus vector, retrovirus vector and the like. Commercially available vectors may include pUC19, pTV118N (Takara Shuzo Co., Ltd.), pUEX2 (Amersham), pGEX-4T, pKK233-2 (Pharmacia), pMAM-neo (Clontech), pGL2 (Promega), pDNA3.1+ (Invitrogen) and the like. A method of constructing such vectors can be performed utilizing restriction enzyme, ligase and the like by the conventional method without any particular limitation.

In the case where bacteria, in particular E. coli, are used as a host cell that is transformed or transfected with the above described vector, the vector may usually comprise at least a promoter region (containing promoter, operator and Shine-Dalgarno sequence), initiation codon, a nucleotide sequence encoding the novel collectin, termination codon, terminator region and the like. In the case where yeast or an animal cells are used as a host, it is preferable that the vector includes at least promoter, initiation codon, nucleotide sequences encoding a signal peptide and the novel collectin, and termination codon. Moreover, enhancer sequence, 5'- and 3'-untranslated region of the novel collectin, splicing junction, polyadenylation site and selectable marker can be also inserted into the vector.

As a promoter incorporated into a vector of the present invention, conventionally well known SV40 (Simian Virus 40), SRα, cytomegalovirus (CMV) promoter, actin promoter, viral LTR (Long Terminal Repeat), for example, HTLV-1 LTR, HIV-LTR, Rous sarcoma virus LTR, herpes simplex tyrosine kinase virus promoter and the like can be used irrespective of whether it is a strong promoter or a weak promoter.

In the case where the expression is expected in eukaryotic cells of many cell types ranging from normal cells such as fibroblast, nerve cell, blood cell, parenchymal cell and the like to cancer cells, generally employed promoter may include cytomegalovirus, thymidine kinase (TK), β-actin and SV40 early gene promoter and the like. Since an enhancer is usually combined with a promoter sequence, it can be utilized as it is.

Moreover, in the case where cells and tissues in which the gene is introduced and expressed have been specified, a promoter specific for the cell can be selected. Furthermore, by combining these promoters in a homologous or heterologous manner, high expression can be expected, and an effect may be exerted that the novel collectin is stably obtained.

On the other hand, a promoter used in prokaryotic cells, PBAD, PL, trc, T7, SP6, T3, lac and the like may be included.

In principle, a promoter that can result in high expression of the novel collectin in the cells from prokaryotic cells can be appropriately selected and used as a promoter of the present invention, as long as the promoter is compatible with the host. Moreover, in the case where cells and tissues in which the gene is introduced and expressed have been specified, a promoter specific for the cell can be selected. Furthermore, by combining these promoters in a homologous or heterologous manner, high expression can be expected, and an effect may be exerted that the novel collectin is stably obtained.

On the other hand, promoter used in yeast may include GAL1, AOX1, CUP1, PGK and the like.

A selectable marker incorporated in the above described vector for recovering only the intended vectors may include dihydrofolate reductase (hereinafter, abbreviated as DHFR) gene, methotrexate resistant gene, neo gene (G418 resistant gene) and the like, and for example, in the case where DHFR gene is used as a selectable marker employing CHO cell of which DHFR gene is deleted, the selection can be also performed by a thymidine free medium. In addition, when the gene is expressed in the cell by culturing in the medium at a higher methotrexate concentration and selecting resistant cells, the cell strain with higher expression can be also obtained.

The present invention also contemplates a host cell transformed or transfected with the vector thus constructed, which can express the novel collectin of the present invention, for example, animal cells, insect cells and microorganic cells (E.coli, Bacillus subtilis, yeast, and the like).

Animal cells used as the host cell of the present invention may include cells derived from human, but not limited thereto. For example, CHO cell, COS cell, BHK cell, Vero cell, myeloma cell, HEK 293 cell, HeLa cell, Jurkat cell, mouse L cell, mouse C127 cell, mouse FM3A cell, mouse fibroblast, osteoblast, cartilage cell and the like may be included. Moreover, as the microorganic cell, for example, bacteria such as E. coli, Bacillus subtilis and the like, and yeasts such as *Saccharomyces cerevisiae,* beer yeast and the like may be included.

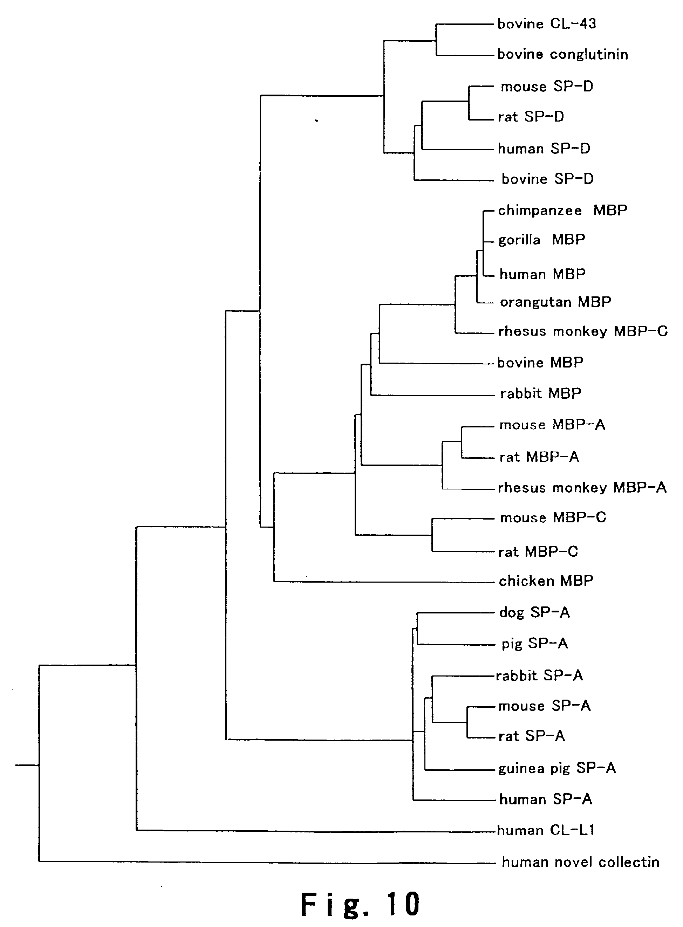

Moreover, according to the present invention, a probe for screening a novel collectin related molecular species including the above polynucleotide is provided Fragments may alternatively be used The novel collectin related molecular species means a molecular species of a novel collectin having a characteristic structure as shown in FIG. 1, without being included in the known collectins shown in FIG. 10. Using a probe of the present invention, a desired novel collectin related molecular species can be acquired by examination of a hybridizing ability between the probe and a gene obtained from tissue cells and the like of a variety of organisms, screening a gene encoding the desired novel collectin related molecular species through searching a gene that can hybridize with the probe under a relatively stringent condition. Examples of fragments of the polypeptide, those comprising a nucleotide sequence of at least a stretch of 10 bases or more, preferably a stretch of 20 bases or more out of a sequence of 1291-1698 in SEQ ID NO: 1 that comprises a carbohydrate recognition domain structural region, or those comprising a nucleotide sequence of at least a stretch of 10 bases or more, preferably a stretch of 20 bases or more out of a sequence of 796-1236 in SEQ ID NO: 1 that comprises a collagen-like region may be useful as a polynucleotide for constituting the probe in terms of its specificity.

An antibody that recognizes a novel collectin and/or derived molecules obtained according to the present invention can also be made. As referred to herein, the term "derived molecules" may include fragments having similar characteristics with the novel collectin (for example, fragments containing carbohydrate recognition domain and/or collagen-like region), precursors of the novel collectin, and polypeptides comprising amino acid sequence encoded by the nucleic acid which can hybridize under the stringent condition with the nucleotide sequence coding the fragments, precursors, and/or complementary nucleic acid sequence thereto.

An antibody (for example, polyclonal antibody, monoclonal antibody and peptide antibody) or anti-serum for the novel collectin and/or derived molecules can be manufactured using the present novel collectin and/or derived molecules, or fusion protein containing the same, and the like, as an antigen in accordance with known method of generating the antibody or anti-serum. Namely, the present novel collectin and/or derived molecules, or fusion protein containing the same may be administered as an antigen alone or in combination with a diluent or a carrier to the site in which antibody production is allowed through the administration to the warm-blooded animals. Upon administration, Freund's complete adjuvant or Freund's incomplete adjuvant may be administered in order to elevate the production of the antibody. The administration may be usually performed once per every 1-6 weeks, 2-10 times in total. The warm-blooded animals employed may include for example, monkey, rabbit, dog, guinea pig, mouse, rat, sheep, goat, chicken and the like, and mouse and rat are preferred. Among rats, Wistar and SD strain rat and the like are preferable, and among mice, BALB/c , C57BL/6 and ICR strain mouse and the like are preferably used.

When the monoclonal antibody producing cells are prepared, an individual that is found with antibody titer is selected from the warm-blooded animal e.g., mouse, then spleen or lymph node is recovered in 2-5 days after the final immunization, followed by fusion of myeloma cells and the antibody producing cells contained therein to yield the monoclonal antibody producing hybridoma. The measurement of the antibody titer in anti-serum may be performed by, for example, the reaction of the anti-serum with a labeled novel collectin described below or the novel collectin, and thereafter determining the activity of the labeling agent coupled to the antibody. An operation for fusion can be carried out according to the known methods, for example, Kohler and Milstein's method (*Nature,* 256: 495, 1975) and a modified method thereof (*J. Immunol. Method.* 39: 285, 1980; *Eur. J. Biochem.,* 118: 437, 1981; *Nature,* 285: 446, 1980). As agents for promoting fusion, polyethylene glycol (PEG), Sendai virus and the like, preferably PEG may be employed. In order to further enhance fusion efficiency, lectin, poly-L-lysine or DMSO may be added ad libitum.

Myeloma cells may include for example, X-63Ag8, NS-1, P3U1, SP2/0, AP-1 cells and the like, however, SP2/0 cells may be preferably used. The suitable rate between the number of antibody producing cells (splenocyrte) and the number of myeloma cells, which are used, may be 1:20-20:1, and cell fusion can be efficiently carried out by adding PEG (preferably PEG 1000-PEG 6000) at a concentration of approximately 10-80%, and incubating for 1-10 minutes at 20-40°C, preferably at 30-37°C. Although a variety of methods can be utilized for screening a hybridoma producing the antibody to the novel collectin and/or derived molecules, for example: a method in which hybridoma culture supernatant is added to a solid-phase (for example, microplate) where the novel collectin antigen is directly absorbed or along with a carrier, next, an anti-immunoglobulin antibody labeled by a radioactive substance, enzyme or the like, or protein A is added thereto, and the anti-novel collectin antibody coupled to the solid-phase is detected; a method in which hybridoma culture supernatant is added to the solid-phase where anti-immunoglobulin antibody or protein A is absorbed, followed by addition of the novel collectin labeled with a radioactive substance, enzyme or the like, and detection of the anti-novel collectin monoclonal antibody coupled to the solid-phase; and the like may be adopted.

Selection and cloning of the monoclonal antibody to the novel collectin and/or derived molecules may be performed according to a method known in the art or a method conformed thereto. In general, they may be performed in a selective medium for use in animal cells to which HAT (hypoxantine, aminopterin and thymidine) are added. As the medium for selection, cloning and growing, any medium may be employed as long as the hybridoma can grow therein. For example, RPMI medium containing 1-20%, preferably 10-20% of fetal bovine serum, GIT medium containing 1-10% of fetal bovine serum, a serum-free medium for use in hybridoma culture or the like can be employed. The temperature for culture may be preferably about 37°C. The time period of culture is usually, from 5 days to 3 weeks, preferably, from 1 week to 2 weeks. The culture is generally performed under the presence of air containing 5% carbon dioxide gas. The antibody titer of hybridoma culture supernatant can be measured in a similar manner to the procedure of the above-described measurement of the anti-novel collectin antibody titer in an the anti-serum. Specifically, as the method for measurement, radioimmunoassay (RIA) method, enzyme-linked immunosorbent assay (ELISA) method, fluorescence immunoassay (FIA) method, plaque assay method, coagulation reaction method and the like can be employed, however, the following ELISA method described below may be preferable.

A protein prepared by a similar procedure as in the case for immune antigen is immobilized on the surface of each well of ELISA plate. Next, for the purpose of preventing non-specific absorption, bovine serum albumin (BSA), mouse serum albumin (MSA), ovoalbumin (OVA), gelatin, skim milk or the like are immobilized. Hybridoma culture supernatant is added to these respective wells, allowed to stand for a certain time period to effect immunoreaction. Each of the wells is washed using phosphate buffered saline (PBS) or the like as a wash solution. It is preferable to add a surface- active agent to the wash solution. An enzyme-labeled secondary antibody is added and allowed to stand for a certain time period. As the labeling enzyme, β-galactosidase, alkaline phosphatase, peroxidase and the like can be employed. After each well is washed with the same wash solution, a substrate solution for the used lebeling enzyme is added, and enzyme reaction is effected. In the case where the intended antibody exists in the added hybridoma culture supernatant, the enzyme reaction is proceeded, and the color of the substrate solution is changed.

Cloning can be generally performed by a semi-solid agar method, a limiting dilution culture method and the like which are known in the art, in brief, after wells where the intended antibody is produced are determined, cloning is performed to obtain a single clone. As for a cloning method, the limiting dilution culture method may be utilized wherein the hybridoma cells are diluted and cultured thereby forming one colony per well of culture plate. For cloning by the limiting dilution culture method, feeder cells may be employed, or cell growth factor such as interleukin 6 may be added in order to enhance colony forming ability. Alternatively, cloning can be performed using FACS (fluorescence-activated cell sorter) and a single cell manipulation method. The cloned hybridoma may be preferably cultured in a serum-free medium, and the optimal amount of the antibody is added to the supernatant. A single hybridoma thus obtained may be cultured in a large scale using flasks or a cell culture equipment, or otherwise, intraperitoneal culture in an animal is performed (*J. Immunol. Meth.,* 53:313, 1982), to allow for obtaining the monoclonal antibody. Culturing of the cells within a flask can be conducted using medium for cell culture (IMDM, DMEM, RPMI 1640, MEM and the like) containing 0-20% of fetal calf serum (FCS). When intraperitoneal culture in an animal is carried out, the animal of the same species, the same strain with the animal from which myeloma cell used for cell fusion is derived, athymic nude mouse or the like may be preferably used, and the hybridoma is transplanted after the previous administration of mineral oils such as pristan and the like. After 1-2 weeks, myeloma cells are grown in peritoneal, and ascites fluid containing the monoclonal antibody can be recovered.

A monoclonal antibody may be obtained as the one not cross-reacted with the other proteins by selecting the one that recognizes an epitope specific for the novel collectin and/or derived molecules. In general, an epitope presented by at least three or more serial amino acid residues, desirably amino acid sequence of 7-20 amino acids from the amino acid sequence constituting a protein is reported to show an epitope that is inherent in the protein. Therefore, a monoclonal antibody that recognizes an epitope constituted from the peptides selected from the amino acid sequences set forth in either of the novel collectin and/or derived molecules, and have amino acid sequences consisted of at least three serial amino acid residues may be referred to as a monoclonal antibody specific for a novel collectin and/or derived molecules of the present invention. When an amino acid sequence conserved between amino acid sequences set forth in the novel collectin and/or derived molecules is selected, a common epitope for the novel collectin family can be selected. Alternatively, when a region containing an amino acid sequence specific for the respective sequence is selected, a monoclonal antibody capable of distinguishing respective proteins can be selected.

Isolation and purification of a monoclonal antibody to the novel collectins and/or derived molecules can be performed according to the isolation and purification method of immunoglobulin, similarly to the cases of the general polyclonal antibodies. As known purification methods, for example, salting-out method, alcohol precipitation method; isoelectric point precipitation method, electrophoresis method, ammonium sulfate precipitation method, absorption-desorption method by ion-exchanger (e.g., DEAE (diethylaminoethyl) Sepharose and the like), ultracentrifuge method, gel filtration method, a process of specific purification by collecting only an antibody with active absorbent such as antigen binding solid-phase, protein A, protein G or the like and dissociating the binding to obtain the antibody can be carried out. For the purpose of preventing the aggregate formation and reduction of the antibody titer during the purification process, for example, human serum albumin is added at a concentration of 0.05-2%. Besides, amino acids such as glycine, α-alanine and the like, particularly, basic amino acids such as lysine, arginine, histidine and the like, sugars such as glucose, mannitol and the like or salts such as sodium chloride and the like may be added. IgM antibody is known to be especially liable to aggregate, therefore, treatment with β-propionolacton and acetic anhydride may be conducted.

A polyclonal antibody can be manufactured according to the methods known in the art or methods conformed thereto. For example, immune antigen (protein antigen) itself, or a prepared complex thereof with a carrier protein, may be used to immunize a warm-blooded animal similarly to the above-described method of producing a monoclonal antibody, accordingly, the monoclonal antibody can be generated through collecting a material which contains an antibody to a protein or its fragments of the present invention from the immunized animal, and through isolation and purification of the antibody. In respect to the complex of the immune antigen and the carrier protein used for immunizing the warm-blooded animal, as for kind of the carrier protein as well as the mixing rate of the carrier and hapten, any kind of carrier protein may be crosslinked at any mixing ratio of the carrier and hapten as long as the antibody can be efficiently generated for the hapten crosslinked and immunized with the carrier. However, a method may be employed in which bovine serum albumin, bovine thyroglobulin, hemocyanin or the like is coupled with hapten at a weight ratio of about 0.1-20, preferably about 1-5 per hapten 1. Moreover, a variety of condensing agents can be used for coupling hapten and carrier, however, glutaraldehyde or carbodiimide, maleimide active esters, active ester reagents containing thiol group or dithiopyridyl group and the like may be employed. The condensing product may be administered itself alone or in combination with a carrier or a diluent to a warm-blooded animal. Upon administration, Freund's complete adjuvant or Freund's incomplete adjuvant may be administered in order to enhance the antibody producing ability. The administration may be performed usually once every 2-6 weeks, about 3-10 times in total. A polyclonal antibody can be collected from the blood, ascites fluid and the like, preferably from blood of the warm-blooded animal that has been immunized by the above-described method. The measurement of the polyclonal antibody titer in the anti-serum can be performed similarly to the measurement of the antibody titer of the above-described serum. The isolation and purification of the polyclonal antibody can be performed according to the isolation and purification method of immunoglobulin, similarly to the above-described isolation and purification method of a monoclonal antibody.

Moreover, an antibody can be generated by using a fusion protein comprising the novel collectin and/or derived molecules and another protein or a polypeptide fragment as an immunogen. As another protein or the polypeptide constituting such a fusion protein, GST (glutathione-S-transferase) may be particularly suitable, besides, polyhistidine (preferably 6 histidine residues), calmodulin binding peptide (CBP), protein A and the like may be employed.

When polyhistidine is employed, absorption to nickel-chelating resin can be utilized for isolation and purification of the fusion protein expressed by a gene recombinant process, wherein addition of EDTA or imidazole substance as well as pH change may be adopted for dissociating the protein from the resin. When CBP is employed, the expressed fusion protein may be subjected to an affinity chromatography using calmodulin affinity resin, and then may be dissociated from the resin by adding EGTA. Further, when protein A is employed, the expressed fusion protein may be subjected to an affinity chromatography using IgG sepharose (e.g., IgG Sepharose 6FF), and thereafter may be dissociated from the resin by changing pH.

Moreover, another candidate for the protein or the polypeptide fragment to be employed in the fusion protein may include for example, Xpress, Thioredoxin, c-myc, V5, HA/c-myc and the like, and after the intended fusion protein with the novel collectin is expressed, it can be isolated and purified by an antigen-antibody affinity column using an antibody capable of recognizing such another protein as an epitope.

In the case where a monoclonal antibody is obtained using such fusion protein as an immunogen, the novel collectin may be employed for screening the antibody, however, the screening may be preferably carried out with the fusion protein used as an immunogen in terms of the selectivity.

The aforementioned preferable immunogen, namely a fusion protein comprising GST and a novel collectin and/or derived molecules, may be suitably produced by: culturing *Escherichia coli* or animal cells transformed or transfected with an expression vector comprising a coding region of GST gene and a coding region of a novel collectin and/or derived molecules; and then isolating the fusion protein from the culture fluid and/or cells. Thus obtained fusion protein may be preferably purified further based on an affinity to a support carrying glutathione, e.g., glutathione sepharose beads, and a method of eluting the fusion protein from the glutathione sepharose beads can be performed by the known method. Accordingly, the purified fusion protein that is preferable as an intended immunogen can be obtained.

In a method of preparing a monoclonal antibody using such a fusion protein as an immunogen, a novel collectin and/or derived molecules may be employed for screening the antibody, however, it is more preferable to perform the screening using the fusion protein as an immunogen in terms of the specificity.

The monoclonal antibody and polyclonal antibody to the novel collectin and/or derived molecules can be utilized for diagnosis and treatment of diseases related to the novel collectin. It should be noted that in the case where the antibody is contemplated to administer particularly to a human body, the antibody such as humanized antibody or chimeric antibody, having no immunogenicity to human or having compromised immunogenicity as far as possible may be preferably used. When a mouse monoclonal antibody is administered into a human body, there may be several risks such that a variety of side effects may occur, because it is a heterologous protein to human. Hence, human monoclonal antibody would be desirable, however, fusion efficiency was poor, and to obtain a hybridoma that is stably producing the antibody was difficult. However, the technologies have been progressed so far, thus, generation of human monoclonal antibodies or chimeric antibodies has been possible at present.

The term "chimeric antibody" is referred to as a chimeric molecule of a mouse antibody and a human antibody. It is ethically impossible to manufacture an antibody by immunizing a given antigen to a human body. Therefore, a mouse is immunized, then a variable region (V region) of an antibody which may bind to an antigen is cut out from a gene of the mouse monoclonal antibody, and a chimeric gene is prepared by coupling it to a constant region (C region) of an antibody gene derived from human myeloma cells. When this chimeric gene is expressed in a host cell, human/ mouse monoclonal antibody can be produced. Since a chimeric antibody has low antigenicity to human, it can be utilized as a monoclonal antibody to be administered into a human body for treatment, for diagnostic imaging and the like. With respect to known related technologies of chimeric antibodies, see, Japanese Unexamined Patent Publication No. H05-304989 (1993), Japanese Unexamined Patent Publication No. H04-330295 (1992), W09106649, Japanese Unexamined Patent Publication No. S63-036786 (1988), and Japanese Patent Publication No. H06-98021 (1994) and the like.

However, a humanized antibody, which is reported to be more useful than a chimeric antibody has been recently developed. The term "humanized antibody" is an antibody, which is generated by grafting only a gene sequence of an antigen-binding site (CDR: complementary determining region) of an antibody molecule to a human antibody gene (CDR grafting), thus, the entire molecule is humanized except CDR of the antibody molecule. Since this antibody has less portions of mouse antibody than human/mouse chimeric antibody, it is believed to have less antigenicity and to be safer. In our country, a clinical trial of humanized antibody to adult T cell leukemia has been currently practiced. With respect to methods of manufacturing humanized antibody and related technologies thereof, see, Patent Applications such as Genentech (USA) (WO9222653, WO9845332, WO9404679, W09837200, and WO9404679) and Celltech (UK) (W09429451, WO9429351, WO9413805, WO9306231, WO9201059, WO9116927, WO9116928, WO9109967, WO8901974, and WO8901783) and the like.

Methods of generating a human monoclonal antibody may include: a method wherein transformation is effected with Epstein-Barr virus (EBV), followed by a method of fusing the transformed cells with parent cells; a method of preparing a chimeric antibody or a humanized antibody by utilizing genetic engineering; and the like, in addition to the cell fusion method. The chimeric antibody means an antibody prepared through joining immunoglobulin gene fragments of heterologous animals, and the humanized antibody means an antibody prepared through modification of an antibody of a heterologous antibody to human, such as that from mouse and the like, resulting in substitution of the primary structure with the corresponding primary structure of human except for a complementary determinant region (CDR) of H chain and L chain. As for parent cells for use in the preparation of human monoclonal antibody, SHM-D 33 strain (ATCC CRL 1668) or RF-S1 strain, heteromyeloma of human/mouse, may be suitable employed to achieve a high fusion efficiency comparable to that of mouse parent cells. Thus obtained hybridoma using these parent cells can be subjected to cloning without feeder cells, and can produce an IgG type antibody in a comparatively stable manner at a large amount. ERDF medium to which 15% FCS is added may be employed for the culture of parent cells, and other procedures may be similarly carried out to those of the mouse. Moreover, in order to generate human monoclonal IgG type antibody, it is preferable that human lymphocyte sufficiently sensitized with an antigen is collected from peripheral blood for use.

When it is difficult to obtain lymphocyte that is sufficiently sensitized with an antigen, antigen sensitization *in vitro* can be also carried out. An antibody can be humanized by employing the above-described method and the like, accordingly, the antibody is very useful for administering to a human body.

It is also possible to carry out a method for obtaining a novel collectin related molecular species, or novel collectin and/or derived molecules, comprising a step of screening a protein using the antibody set forth above to obtain the novel collectin related molecular species, or novel collectin and/or derived molecules. In this method, a protein having immunoreactivity is screened using the above-described antibody in order to isolate and obtain a novel collectin related molecular species, or novel collectin and/or derived molecules, which may be contained in a variety of body fluids of organisms, tissue cells and the like. In the screening step, the expression screening of cDNA library may be preferably utilized.

Methods of quantitative determination of a novel collectin and/or derived molecules, comprising measuring an amount of the novel collectin and/or derived molecules contained in a test sample using the antibody set forth above, on the basis of the immunological binding property thereof can also be performed.

In this method, in order to measure an amount of the novel collectin and/or derived molecules, the above-described antibody may be employed in immunoblotting, immunoprecipitation, ELISA or the like, and in particular, ELISA method that is convenient may be preferably performed. In addition, a sandwich method comprising a step of detecting a sandwich complex generated by reacting a novel collectin and/or derived molecules with an antibody and labeled antibody coupled to, for example, an insoluble support, otherwise, a competitive method comprising steps of: competitively reacting a labeled novel collectin and/or derived molecules with a novel collectin and/or derived molecules in a test sample with an antibody; and measuring the novel collectin and/or derived molecules in the sample based on the amount of the antigen reacted with the antibody may be utilized to determine the amount of a novel collectin and/or derived molecules.

Upon determination of an amount of a novel collectin and/or derived molecules by the sandwich method, a two steps method, wherein an immunoreaction of an immobilized antibody with a novel collectin and/or derived molecules is allowed first, then unreacted substances are completely removed by washing, followed by addition of a labeled antibody; or a one step method, wherein an immobilized antibody, a labeled antibody, and a novel collectin and/or derived molecules are simultaneously mixed may be performed.

Insoluble support used for such determination may include for example, synthetic resin such as polystyrene, polyethylene, polypropylene, polychlorinated vinyl, polyester, polyacrylate ester, nylon, polyacetal, fluorine contained resin and the like; polysaccharides such as cellulose, agarose and the like; glass; and metal etc. The insoluble support may be in several forms for example, tray-like, spherical, fibrous, cylindrical, discal, vessel-like, cell-like, tubular and the like. The support with the antibody being absorbed may be stored in a cold place, in the presence of an antiseptic agent such as sodium azide.

For immobilization of an antibody, a known chemical binding method or a physical absorption method may be adopted. The chemical binding method may include for example, glutaraldehyde-utilizing method, maleimide method, wherein N-succinimidyl-4-(N-maleimidemethyl) cyclohexane-1-carboxylate, N-succinimidyl-2-maleimideacetate or the like may be used, carbodiimide method, wherein 1-ethyl-3-(3-dimethylaminopropyl) carbodiimide hydrochloride may be used. In addition, maleimidebenzoyl-N-hydroxysuccinimide ester method, N-succinimidyl-3-(2-pyridylthio) propionic acid method, bisdiazotized benzidine method, dipalmityllysine method may be included. Alternatively, a complex which was previously formed by a reaction of a test detected material with an antibody of which epitope is in a different kind, may also be captured after the third antibody to said antibody is immobilized according to the method as mentioned above.

The material to be used for labelling the antibody may be enzyme, fluorescent material, luminescence material, radioactive material, metal chelate or the like. An enzyme may include for example, peroxidase, alkaline phosphatase, β-D-galactosidase, malate dehydrogenase, staphylococcus nuclease, delta-5-steroid isomerase, α-glycerolphosphate dehydrogenase, triose phosphate isomerase, horseradish peroxidase, asparaginase, glucose oxidase, ribonuclease, urease, catalase, glucose-6-phosphate dehydrogenase, glucoamylase, acetylcholine esterase, and the like; and fluorescent material may include for example, fluorescein isothiocyanate, phycobilin protein, rhodamine, phycoerythrin, phycocyanin, allophycocyanin, orthophthalic aldehyde and the like; luminescence material may include isoluminol, lucigenin, luminol, aromatic acridiniumester, imidazole, acridinium salt and modified ester thereof, luciferin, luciferase, aequorin and the like; and radioactive material may include ¹²⁵I, ¹²⁷I, ¹³¹I, ¹⁴C, ³H, ³²P, ³⁵S and the like, but not limited thereto as long as the material can be used in immunological determination methods. Further, low molecular weight hapten such as biotin, dinitrophenyl, pyridoxal or fluorescamine may be conjugated to the antibody. Preferably horseradish peroxidase may be used as a labelling enzyme. This enzyme can react with a lot of substrates, while being readily conjugated to the antibody by a periodic acid method.

When an enzyme is used as a labelling material, a substrate for measuring its activity, and a color-developing agent are employed. When peroxidase is used as an enzyme, H₂O₂ may be used as a substrate solution, and 2,2'-azino-di-[3-ethylbenzthiazolin sulfonate] ammonium (ABTS), 5-aminosalicylic acid, orthophenylenediamine, 4-aminoantipyrine, 3,3',5,5'-tetramethylbenzidine or the like may be used as a color-developing agent; when alkaline phosphatase is employed as an enzyme, the substrate may be orthophenylphosphate, paranitrophenylphosphate or the like; alternatively, when β-D-galactosidase is used as an enzyme, the substrate may be fluorescein-di-(β-D-galactopyranoside), 4-methyl-umbelliferyl-D-galactopyranoside, or the like.

As a crosslinking agent, N,N'-orthophenylenedimaleimide, 4-(N-maleimidemethyl) cyclohexanoyl N-succinimide ester, 6-maleimidehexanoyl N-succinimide ester, 4,4'-dithiopyridine, or other known crosslinking agents can be utilized. The reaction of such a crosslinking agent with the enzyme and the antibody may be proceeded in accordance with known methods depending upon the properties of the crosslinking agent that was employed.

Additionally, the antibodies to be used may be any fragments of the antibodies for example, Fab', Fab, F(ab')₂ depending on conditions. Furthermore, enzymatically labelled antibody may be prepared by using similar process as any one of polyclonal antibodies and monoclonal antibodies. When the enzymatically labelled antibody that was obtained by using the aforementioned crosslinking agent is purified by any known methods, more sensitive immunological determination system can be achieved. The enzymatically labelled antibody that was purified in such a manner may be mixed with a stabilizer such as thimerosal, glycerol or the like, alternatively, may be lyophilized, and then stored in a cold and dark place.

Test samples for measurement in the above quantitative determination are not limited as far as they are the samples containing a novel collectin and/or derived molecules, or the samples containing a precursor thereof, which may include body fluids such as plasma, serum, blood, urine, tissue fluid, cerebrospinal fluid and the like.

Furthermore, a kit for quantitative determination of a novel collectin and/or derived molecules, comprising the antibody as mentioned above, wherein an amount of the novel collectin and/or derived molecules in a test sample is measured by ELISA method using this antibody can be made. The kit further comprises regents required for the quantitative determination as described above besides the antibody.

A method for producing a novel collectin and/or derived molecules, comprising a step of isolating a novel collectin and/or derived molecules using the antibody as above-described can also be performed. In order to isolate the novel collectin and/or derived molecules in such a method, affinity chromatography and/or immunoprecipitation may be carried out by a column method, a batch method or the like which comprises the steps of: allowing specific interaction between antigen and antibody by contacting a sample with a support coupled to the above-described antibody; washing the support; and eluting the coupled protein intended.

The sample in this method may be for example, the one obtained through preparing from placenta, spleen and the like derived from human, or the one prepared by expression from a polynucleotide encoding the above-described novel collectin of the present invention, however, expression product may be preferably used, since it can be stably supplied as the sample, accordingly, a method comprising such an expression step may be contemplated in the present invention.

Moreover, further aspect contemplated by the present invention is a method for identifying the presence of a novel collectin and/or derived molecules derived molecules within tissues, wherein immunohistological examination is conducted using the above-described antibody. The immunohistological examination may be for example, techniques such as *in situ* immunohistological staining using a labeled antibody, in which a novel collectin and/or derived molecules may be detected.

Furthermore, in accordance with the present invention, a transgenic non-human animal that is stably introduced with a recombinant gene comprising the above-described polynucleotide according to the present invention into the chromosome, and that can express a novel collectin is provided. A transgenic non-human animal wherein introduction of a gene is effected in a non-human animal comprising a homologue of the novel collectin according to the present invention, in a manner to modify (for example, so that the expression is inhibited, promoted or expressed under only limited conditions predetermined) the gene expression of the homologue can also be generated. In this non-human animal, the expression level of the relevant gene can be artificially modified to be elevated or lowered in comparison to the original gene expression level by eliciting mutations such as deletion, substitution, addition and/or insertion in part of several important sites (enhancer, promoter, intron and the like) that are normally regulating the gene expression. The introduction of the mutations can be carried out by the known methods. (e.g., Lesley, S.A. et al., *Promega Notes Magazine,* **46,** 6-10, 1994; Kunkel, T.A. et al., *Methods Enzymol.,* **154,** 367-382, 1987; Sayers, J.R. et al., *Biotechniques,* **13,** 592-596, 1992; Ito W., et al., *Gene,* **102,** 67-70, 1991; Cormack, B., *Current Protocols in Molecular Biology,* 8.5.1-8.5.9, 1987). In this method, the gene may include cDNA, genomic DNA or synthetic DNA. Moreover, the expression of a gene includes both of the steps of transcription and translation.

Transgenic non-human animals as described above may be useful for development of disease model animals for studies on functions, or regulation of expression of the novel collectin and/or the derived molecules, elucidation of the mechanisms involved in the diseases in which the novel collectin and/or derived molecules are expected to participate, and for use in screening and safety test for pharmaceutical products.

The term "transgenic animal" means in its narrow definition, an animal in which an exogenous gene is artificially introduced into germ cells by gene recombination, while in its broader definition, "transgenic animal" means an animal in which an exogenous gene is stably introduced into chromosomes in an early stage of development of the individual, thus transmission to the descendant thereof can be resulted as an inherited characteristic, including antisense transgenic animals in which a function of a specified gene is suppressed using antisense RNA, animals in which a specific gene is knocked out using embryonic stem cells (ES cell), and animals in which point mutated DNA is introduced.

As used herein, "transgenic animal" may be construed in its broader sense, including all vertebrate animals except human. A transgenic animal as described above may be useful for development of disease model animals for studies on functions, or regulation of expression of the novel collectin and/or the derived molecules, elucidation of the mechanisms involved in the diseases related to the cells where those molecules are expressed in human, and for use in screening and safety test for pharmaceutical products.

A method for producing a transgenic animal may include a method in which a gene is directly introduced into a nuclei of the pronucleus stage egg cell using a micropipet under the phase-contrast microscope (micro-injection method, see, US Patent No. 4,873,191), a method in which an embryonic stem cell is used, and the like. Otherwise, a method in which a gene is inserted into a retrovirus vector or a adenovirus vector followed by infection into egg cells; a sperm vector method in which a gene is introduced into an egg cell via a sperm cell, or the like have been developed.

The sperm vector method is a gene recombinant method in which an exogenous gene is attached to a sperm cell or a exogenous gene is incorporated into a sperm cell by electroporation method and the like, followed by introduction by fertilization to an egg cell, resulting in introduction of the exogenous gene (M. Lavitroanoet *et al,* Cell, 57, 717, 1989). Alternatively, site specific gene recombination *in vivo* by cre/lox P recombinase system of bacteriophage P1, FLP reconbinase system of *Saccharomyces cerevisiae,* and the like can be also employed. Moreover, a method of introducing a transgene of the intended protein into non-human animals has been also reported.

A method of producing a transgenic animal by a micro-injection method may be, for example, performed as indicated below.

First, a transgene essentially constituted from a promoter involving in expression regulation, a nucleotide sequence encoding the novel collectin and poly A signal should be prepared. The expression pattern and an amount of expression of a specific molecule may be affected depending upon the promoter activity, and the produced transgenic animals are different between the strains depending on the number of copies of the introduced transgenes and the introduced sites, therefore, the pattern and the amount of the expression should be confirmed between the strains. Since it is revealed that an amount of expression may vary in connection with an untranslated region as well as splicing, an intron sequence that can be previously spliced in advance of the poly A signal may be also introduced. It is important that a gene used for introduction into a fertilized egg should be a gene having a higher purity as much as possible. The animals for use may include a mouse for collecting fertilized eggs (5-6 weeks), a male mouse for fertilization, a pseudopregnant female mouse, a male mouse received vasoligation, and the like.

In order to efficiently obtain a fertilized egg, ovulation may be induced by gonadotropin and the like. The fertilized egg is recovered, and then a gene placed in an injection pipet is injected into a male pronucleus of the egg in a microinjection method. Animals for bringing back the injected egg to an uterine tube (pseudopregnant female mouse and the like) are prepared, and about 10-15 eggs are transplanted per one animal. Subsequently, extraction of a genomic DNA from the top end of the tail, followed by detection of the transgene by Southern blotting method or PCR method, or positive cloning method wherein a marker gene is inserted which is activated only when a homologous recombination resulted, may be carried out to determine if the transgene is introduced in the mice at birth. Furthermore, in order to confirm the expression of the transgene, a transcription product derived from the transgene is detected by Northern blotting method or RT-PCR method. Alternatively, Western blotting method may be performed with a specific antibody to the protein or a fragment thereof.

Furthermore, a knockout non-human animal can be generated, which is obtained by altering a gene encoding a homologue of the above-mentioned novel collectin according to the present invention in a non-human animal comprising the homologue, in a manner to inhibit the expression of the homologue.

The knockout mouse may be treated to deteriorate the function of the gene of the novel collectin and/or derived molecules. The "knockout mouse" means a transgenic mouse in which a given gene is destroyed by homologous recombination technology thus deterioration of its function is resulted. A knockout mouse can be produced by performing homologous recombination using ES cells, and selecting an embryonic stem cell in which one of the allelic genes was modified/destroyed. A chimera mouse having mixed cells derived from the embryonic stem cell and cells derived from an embryo, may be obtained by, for example, injection of an embryonic stem cell with a gene thereof being manipulated, into blastcyst or morula stage of the fertilized egg. When this chimera mouse (chimera is referred to as a single individual mouse formed with somatic cells based on more than two fertilized eggs) is cross fertilized with a normal mouse, a heterozygote mouse having one of the allelic gene is entirely modified/destroyed can be produced. Furthermore, homozygote mouse can be produced by cross fertilizing the heterozygote mice each other.

The "homologous recombination" means recombination that occurs between two genes having the identical or closely similar nucleotide sequences through a gene recombinant mechanism. A PCR method can be used for selecting the cells with the homologous recombination occurred. A polymerase chain reaction may be carried out using a portion of an inserted gene and a portion of a region expected for the insertion, as primers, consequently, the occurrence of the homologous recombination in the cells can be identified where the amplification product was resulted. In addition, when homologous recombination is caused in a gene that is expressed in an embryonic stem cell, a neomycin resistant gene has been previously ligated to the gene introduced, and after introducing the gene, selection can be readily performed by, for example, leading the cells to be neomycin resistant, or by any another known methods or modified methods thereof (see, Capecchi, *Science,* vol. 244, pp. 1288-1299 (1989), and the like).

The present invention will be described in more detail by the following non-limiting illustrative examples. It is intended that the present invention encompasses all modifications and variations which occur to those skilled in the art upon consideration of the disclosures herein, and in particular those embodiments which are within the broadest proper interpretation of the claims and their requirements.

The Examples demonstrate: the search on EST database (Example 1); preparation of the probe for screening (Example 2); screening of cDNA library derived from human placenta (Example 3); sequencing of nucleotide sequence of the novel collectin (Example 4); genomic Southern analysis of the novel collectin (Example 5); Northern analysis of the novel collectin with various human tissues (Example 6); genomic Southern analysis of the novel collectin with tissues from various species of animals (Example 7); and genetic study of the novel collectin (Example 8); generation of monoclonal antibodies specific for the novel collectin (Example 9); ELISA method using the anti-mouse anti-human novel collection antibody (Example 10); generation of antibodies with lowered immunogenicity to human (Example 11); construction of an expression vector pNOW1-hCL-P1 of the novel collectin (Example 12); selection of a clone expressing the novel collectin (Example 13); production of a transgenic mouse (Example 14); and production of a knockout mouse (Example 15).

### Example 1: Search on EST Database

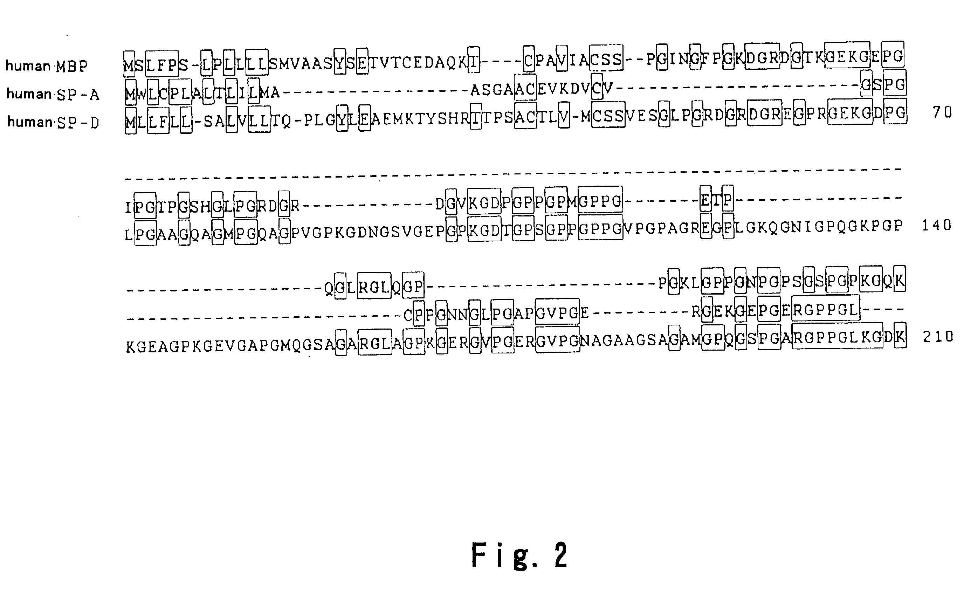

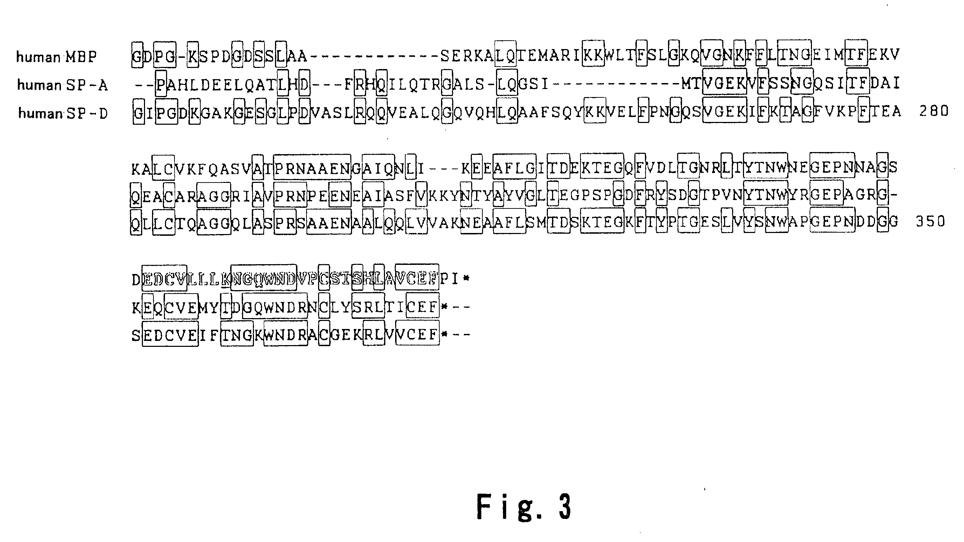

Highly conserved regions between molecules of the known collectins, i.e., human MBP, human SP-A and human SP-D were searched by comparing the amino acid sequences thereof (see Figures 2 and 3, in which amino acid residues which were recognized to be homologous between those proteins were boxed). As a result, it was suggested that the region consisting of 27 amino acids, namely from amino acid 220 to 246 of human MBP sequence (shown in Figure 3, reversed characters), was highly homologous. Therefore, some consensus sequences corresponding to this region were prepared, and searches on EST (Expressed Sequence Tags) database were conducted with such sequences. For this search, the EST database published on October 11, 1996 was employed, which included 676750 sequences.

Consequently, some data comprising highly homologous amino acid sequences with the sequence of the 27 amino acid described above were obtained. Searches on GenBank/EST database with thus obtained data of the amino acid sequences were further conducted, and deduced whether they were derived from known, or unknown genes. Thereby, it was confirmed that data including two highly homologous but unknown nucleotide sequences (registered as: W72977 and R74387) could be obtained when the following amino acid sequence was used as a consensus sequence:
Glu-Lys-Cys-Val-Glu-Met-Tyr-Thr-Asp-Gly-Lys-Trp-Asn-Asp-Arg-Asn-Cys-Leu-Gln-S er-Arg-Leu-Ala-Ile-Cys-Glu-Phe (SEQ ID NO: 3). These were respectively derived from placenta and from fetal heart, and clones indicating portions of nucleotide sequences of the novel collectin.

Thereafter, the clone derived from fetal heart (I.M.A.G.E.Consortium Clone ID 34472) was purchased from ATCC (American Type Culture Collection), and utilized as a screening probe for obtaining a novel collectin.

### Example 2: Preparation of the Probe for Screening

The nucleotide sequence of insert DNA of the clone described above was determined using primers (Pharmacia, M13 Universal Primer (SEQ ID NO: 7, 5'-fluorescein-cgacgttgtaaaacgacggccagt-3') and M13 Reverse Primer (SEQ ID NO: 8, 5'-fluorescein-caggaaacagctatgac-3')).

From thus obtained nucleotide sequence, an open reading flame was selected through matching it to the collectin amino acid sequence, and the nucleotide sequence corresponding to the amino acid sequence which could be read from the above open reading flame was picked out. Then, primers for digoxigenin (DIG) labeled cDNA probes (Reverse Primer: caatctgatgagaaggtgatg (SEQ ID NO: 4) and Forward Primer: acgaggggctggatgggacat (SEQ ID NO: 5)) corresponding to the parts of the nucleotide sequences were produced using DNA/RNA Synthesizer (Applied Biosystems, 392A). DIG labeling was achieved using PCR DIG Probe Synthesis Kit (Boehringer Mannheim). The reaction mixture contained: plasmid DNA (clone W72977, 50 ng/µl), 2 µl (100 ng); 10 x Buffer 5 µl; 25 mM MgCl₂, 5 µl; dNTP (PCR Labeling Mix), 5 µl; 20 µM Reverse Primer, 2.5 µl; 20 µM Forward Primer, 5 µl; H₂O, 28 µl; Taq Polymerase, 0.5 µl. PCR reaction was carried out using Zymoreactor (ATTO Corp.,) through 35 cycles of: 1 minute at 92°C, 1 minute at 55°C, and 2 minutes at 72°C.

### Example 3: Screening of cDNA Library Derived from Human Placenta

First, phage cDNA library derived from human placenta was titrated as follows. Escherichia coli Y1090r⁻, 0.2 ml, which had been cultured at 37°C for 16 hours in mLB medium (LB medium (1 g tryptone, 0.5g yeast extract and 0.5 g NaCl in total volume of 100 ml) containing 10 mM MgSO₄ and 0.2% maltose) and 0.1 ml of cDNA library serially diluted with SM buffer (5.8 g NaCl, 2 g MgSO₄·7H₂O, 2 M Tris-HCl (pH 7.5) 25 ml, and 2% gelatin 5 ml in total volume of 1L) were incubated at 37°C for 15 minutes, then the mixtures were added to 2.5 ml of LB-TOP agarose (0.75% agarose/LB medium) to make homogenous solutions, and plated onto 90 mm *φ* LB Medium Plates (Iwaki Glass), (1.5% agar/LB medium). The added solutions were hardened at room temperature for 15 minutes, then incubated for 5 hours at 42°C. The plaques on each of the plates were counted, and the titer of the phage was calculated. Consequently, the titer calculated to be 2.1 x 10¹⁰ pfu/ml. The screening was performed as follows using the probe prepared in Example 2.

Escherichia coli Y1090r⁻, 0.6 ml, which had been cultured at 37°C for 16 hours in mLB medium, and cDNA library diluted with SM buffer to 1 x 10⁵ pfu were incubated at 37°C for 15 minutes, then the mixture was added to 7.5 ml of LB-TOP agarose (0.75% agarose) to make a homogenous solution. The solution was plated onto ten LB square plates of 140 cm² (Nissui Seiyaku), hardened at room temperature for 15 minutes, then the plates were incubated for 5 hours at 42°C. After plaque formation of each of the plates was confirmed, the transfer to the nylon membranes was performed. The transfer was performed using Nytran 13N (Schleicher and Schuell Co.). The filters (12.5 cm x 9.0 cm in size) were immersed in distilled water for 10 minutes to be wet, then the excess water was removed on Whatmann 3MM Paper, and the filters were placed on the plates having the plaques formed thereon. After standing for two minutes, the filters were recovered and air-dried for 10 minutes. The phage DNA on the filters was denatured for 2 minutes with 0.2 M NaOH/1.5 M NaCl, followed by neutralization with 0.4 M Tris-HCl (pH 7.6) /2 x SSC for 2 minutes and washing with 2 x SSC for 2 minutes. Thereafter, the phage DNA was fixed on the membrane by UV irradiation with GS GENE LINKER (BioRad).

Hybridization, and detection of the signals were conducted as follows. The filters were soaked in 2 x SSC, and the excess moisture was removed using Whatmann 3MM Paper. Then, the filters were placed in a hybridization bag and prehybridization at 68°C for one hour in a hybridization solution (5 x SSC, 1% blocking agent, 0.1% N-lauroyl sarcosine and 0.02% SDS) was performed. Subsequently, the hybridization solution was removed from the bag, and the hybridization solution containing DIG labeled cDNA probe at a concentration of 10 ng/ml was added thereto, and hybridization was proceeded at 55°C for 16 hours. After the hybridization was completed, the filters were washed in a solution of 2 x SSC/0.1% SDS for 5 minutes, 2 times; and further washed in a solution of 0.5 x SSC/0.1% SDS for 15 minutes, 2 times. Then, SDS was removed using DIG buffer I (100 mM Tris-HCl, 150 mM NaCl (pH 7.5)) for 1 minute, and the filters were blocked with DIG buffer II (1% blocking agent in DIG buffer I) for 30 minutes. After washing the filters with DIG buffer I for one minute, a solution of alkaline phosphatase labeled anti-DIG antibody (Boehringer Mannheim) which was diluted to 5000-fold in DIG buffer II was added, and the reaction between antigen and antibody were allowed for 30 minutes at room temperature. The filters were then washed twice with DIG buffer I for 15 minutes at room temperature. Through the subsequent treatment of the filters with DIG buffer III (100 mM Tris-HCl, 100 mM NaCl (pH 9.5), 50 mM MgCl₂) for 3 minutes, the concentration of Mg²⁺ was elevated. A solution of NBT/BCIP (WAKO Chem., Co.) in DIG buffer III was added for color development, accordingly, 10 positive clones were identified.

The plaques corresponding to these clones were excised from the plates and placed in the tubes containing 1 ml of SM buffer. After stirring for 10 minutes, each of the buffer solution was serially diluted with SM buffer, and 0.1 ml of the diluted solution was mixed with 0.2 ml cultures of Escherichia coli Y1090r which had been cultured in mLB medium for 16 hours at 37°C. The mixture was incubated for 15 minutes at 37°C, and then added to 2.5 ml of LB-TOP agarose to make a homogenous solution. The solution was plated into ten 90mm *φ* LB plates, hardened at room temperature for 15 minutes, then the plates were incubated for 5 hours at 42°C. Several plaques were obtained, and the secondary screening was performed essentially in accordance with the procedures of the primary screening.

### Example 4: Sequencing of the Novel Collectin Nucleotide Sequence

The plaque of the clone that was expected as being appropriate among the positive clones obtained in the above secondary screening was excised from the plates, and was placed into a tube containing distilled water 200 µl followed by stirring for 30 minutes at room temperature, then the tube was centrifuged at 15,000 rpm for 5 minutes, and the supernatant was obtained therefrom.

The insert DNA was amplified by PCR with TaKaRa LA PCR Kit Ver.2 (TAKARA Syuzo, Co.) using the resulting supernatant as a template. PCR reactions contained: the supernatant, 27 µl; 10 x LA PCR Buffer II (Mg²⁺ free), 5 µl; 25 mM MgCl₂, 5 µl; dNTP Mix, 8 µl; 20 µM λgt11 Reverse Primer (SEQ ID NO: 9: 5'-ttgacaccagaccaactggtaatg-3'), 2.5 µl; 20 µM λgt11 Forward Primer (SEQ ID NO: 10: 5'-ggtggcgacgactcctggagcccg-3'), 1 µl; LA Taq polymerase, 0.5 µl; and H₂O, to make final volume of 50 µl. PCR reaction was performed using Applied Biosystems Gene Amp PCR System 9600, with 30 cycles of: 20 seconds at 98°C, and 5 minutes at 68°C. The PCR product was verified by the electrophoresis on 1% agarose gel, and purified through excising from the gel. For this purification step, Sephaglas BandPrep Kit (Pharmacia) was used.

The excised DNA fragment was incorporated into pCR2.1 vector (Invitrogen, TA Cloning Kit). The recombinant vector was transformed into TOPIOF' cell included in the Invitrogen TA Cloning Kit. The transformants were cultured in LB medium (containing 100 µg/ml ampicillin), and three kinds of plasmids were extracted by alkaline SDS method.

Thus obtained DNA was cleaved with restriction enzymes that were expected to be adequate, and each DNA fragment was incorporated into pUC18 vector followed by transformation into XL1-Blue cell. The transformants were cultured on LB medium (containing 100 µg/ml ampicillin), and the plasmids were extracted by alkaline SDS method. CL-F1-2-1 resulted in a plasmid containing EcoRI-Hind III fragment and Hind III-EcoRI fragment; CL-P1-3-4 resulted in a plasmid containing EcoRI-BamHI fragment, BamHI-SmaI fragment, SmaI-HindIII fragment, KpnI-Sau3AI fragment, Sau3AI-EcoRI fragment, EcoRI-KpnI fragment and EcoRI-SmaI fragment; CL-P1-3-7 resulted in a plasmid containing EcoRI-BamHI fragment, BamHI-SmaI fragment, SmaI-HindIII fragment, KpnI-Sau3AI fragment, Sau3AI-EcoRI fragment, EcoRI-KpnI fragment and KpnI-EcoRI fragment. The primers prepared were: M13 Universal Primer (SEQ ID NO: 5); M13 Reverse Primer (SEQ ID NO: 6) from the Autoread Sequencing Kit; and the following FITC (Pharmacia, Fluore Prime)-labeled primers that were produced using a DNA/RNA synthesizer followed by sequencing of the nucleotide sequences of the entire regions with Autoread Sequencing Kit (Pharmacia) and A.L.F. Autosequencer.
HPP 1: 5'-fluorescein-cgtgaaaatgaatggaagtgg-3' (SEQ ID NO: 11),
HPP 2: 5'-fluorescein-ttttatccattgctgttcctc-3' (SEQ ID NO: 12),
HPP 3: 5'-fluorescein-ctggcagtccccgaggtccag-3' (SEQ ID NO: 13),
HPP 5: 5'-fluorescein-gctggtccccccggagagcgt-3' (SEQ ID NO: 14)

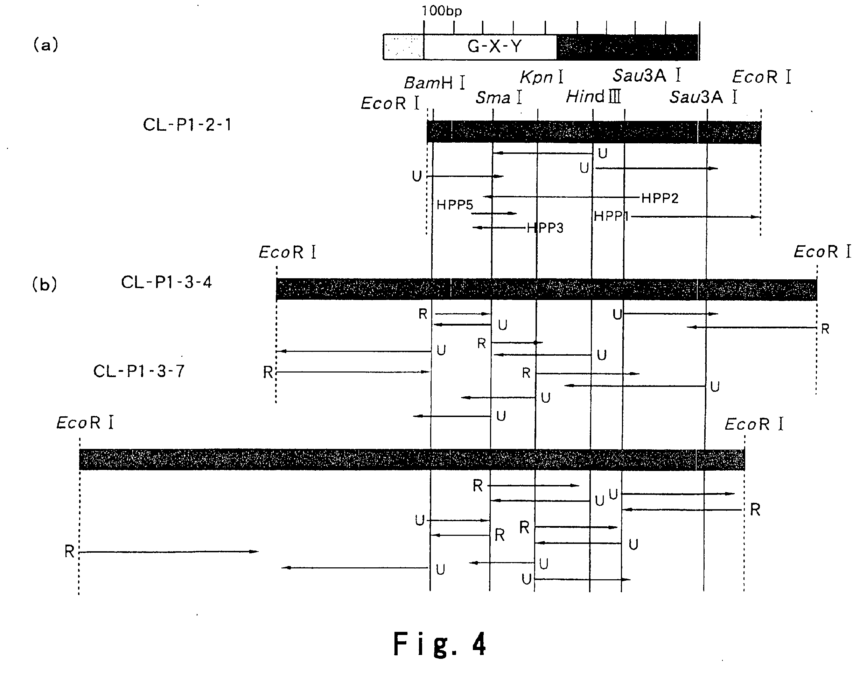

The outline of this nucleotide sequencing strategy performed is shown in Figure 4. An ORF of the obtained collectin is illustrated in Figure 4 (a)" wherein G-X-Y denotes a collagen-like region. Further, in Figure 4 (b), each of the primer names and positions of the nucleotide sequences that were read from the sequencer (shown as allows), as well as M13 Universal Primer (shown as U) and M13 Reverse Primer (shown as R) are illustrated.

In addition, a nucleotide sequence around the 5'-end region comprising a transcription initiation site was determined using Cap site cDNA.

First PCR was performed with Cap Site cDNA, Human Liver (NIPPON GENE) using TGP1 Primer (5'-tcttcagtttccctaatccc-3'(SEQ ID NO: 16)) that was synthesized with the attached 1RC2 Primer (5'-caaggtacgccacagcgtatg-3' (SEQ ID NO: 15)) and 392A DNA/RNA Synthesizer (Applied Biosystems). The employed reaction mixture contained LA PCR Buffer II (Mg²⁺ free), 2.5 mM MgCl₂, each 200 µM of dATP, dCTP, dGTP and dTTP (all of which are manufactured by TAKARA Syuzo, Co.), 1 µl; Cap Site cDNA Human Liver; 0.5 *µ* µ 1RC2 Primer (both of which are manufactured by NIPPON GENE), and 0.5 µM TGP1 Primer, in total volume of 50 µl. PCR was performed using a program comprising 35 cycles of: heat denaturation for 20 seconds at 95°C, anealing for 20 seconds at 60 °C, extension for 20 seconds at 72 °C, with heat denaturation for 5 minutes at 95°C prior to the repeated reaction and final extension for 10 minutes at 72°C. After completing the first PCR, nested PCR was conducted. The reaction was performed using 1 µl of the first PCR product as a template, with primers 2RC2 Primer (5'-gtacgccacagcgtatgatgc-3' (SEQ ID NO: 17)) attached and synthetic TGP2 Primer (5'-cattcttgacaaacttcatag-3' (SEQ ID NO: 18)) that was synthesized similarly to TGP1 Primer, and with the same reaction components and program (except that the cycle number was 25) as in the first PCR. The PCR reaction was performed with TaKaRa PCR Thermal Cycler 480. After thus obtained PCR product was confirmed on agarose gel electrophoresis, the band was excised from the gel, followed by freezing at -80°C for 10 minutes, centrifuge at 15000 rpm, for 10 minutes, and then the supernatant was purified by ethanol precipitation.

The purified DNA fragment was incorporated into pT7Blue Vector (Novagen), and the vector was transformed into competent XL1-Blue cell. The transformants were cultured on LB medium (containing 100 µg/ml ampicillin), and the plasmids were extracted by alkaline SDS method, followed by sequencing of the nucleotide sequence with Autoread Sequencing Kit (Pharmacia) and A.L.F. DNA Sequencer. The employed primers were M13 Universal Primer (SEQ ID NO: 7) and M13 Reverse Primer (SEQ ID NO: 8) attached to AutoRead Sequencing Kit.

As a result, it was confirmed that the cDNA clone of the novel collectin that was obtained in Example 3 contained 2024 bases comprising ORF (open reading frame) of 1026 bases encoding 342 amino acids set out in SEQ ID NO: 2.

Next, the results of homology searches of the DNA and amino acid sequence on GenBank database revealed that the amino acid sequence of the obtained protein is distinct from those of the collectins identified previously and it derives from a novel protein.

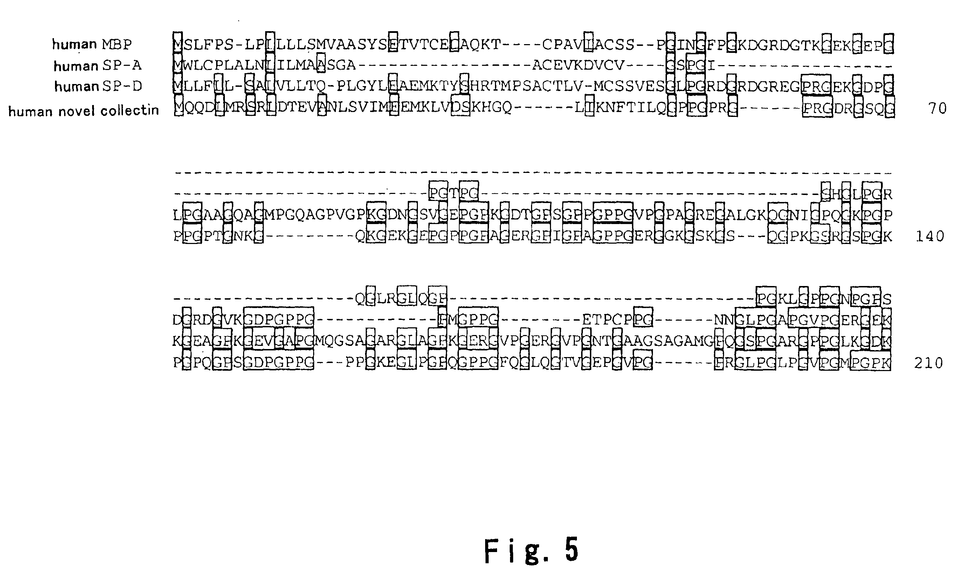

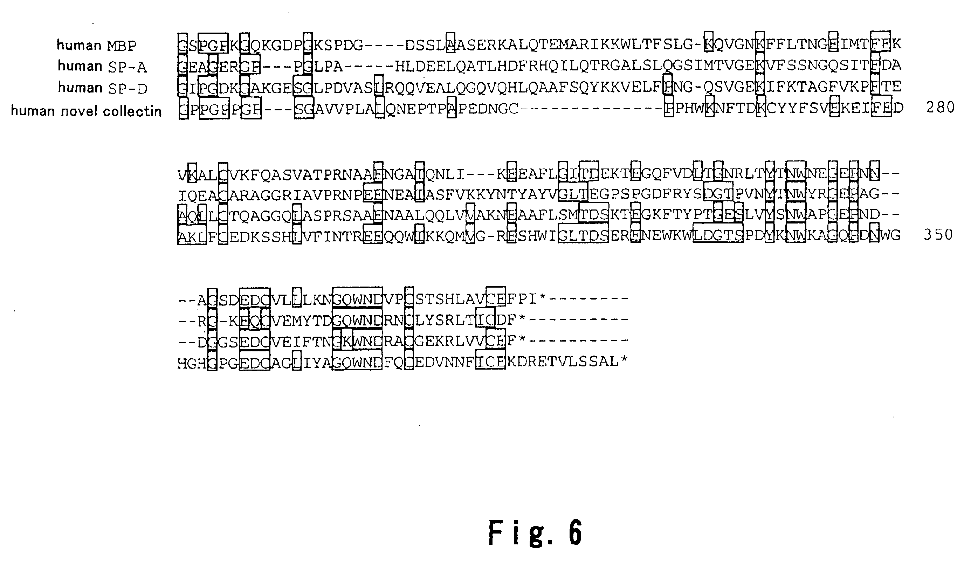

In addition, the amino acid sequence of the novel collectin of the present invention was compared to those of three collectin proteins reported heretofore. The alignment is shown in Figures 5 and 6. Similarly to Figures 2 and 3, homologous amino acid residues were boxed. This alignment suggests that the obtained novel protein shares homology with known collectins and it belongs to the collectin family.

### Example 5: Genomic Southern Analysis of the Novel Collectin

Genomic Southern analysis was performed in order to clarify whether the novel collectin gene comprising the cDNA sequence verified in Example 4 was a single copy gene or a multi copy gene.

Four µg of genomic DNA (Promega) derived from human blood was digested with any one of the restriction enzymes, (1) EcoRI, (2) XbaI, (3) HindIII, (4) PstI, (5) BglII or (6) BamHI, followed by electrophoresis on 0.8% agarose gel at 100 mA, for 3 hours. After the electrophoresis was completed, the DNA was transferred to a nylon membrane (Nytran 13N) to prepare a membrane for the analysis.

For the transfer step, the electrophoresed gel was first immersed in 100 ml of 0.25 N HCl for 10 minutes, washed three times with distilled water, then immersed twice in 100 ml of a denaturalizing solution (1.5 M NaCl, 0.5 M NaOH) for 15 minutes, and immersed in 100 ml of a neutralizing solution (0.5 M Tris-HCl, 3 M NaCl (pH 6.8)) for 30 minutes so that depurination, denaturation and neutralization were accomplished. Thereafter, the DNA was transferred using Vacuum Blotting System (Toyobo Engineering, VB-30). In this step, the membrane which had been pretreated by immersing in 2 x SSC for 5 minutes and in 20 x SSC for 5 minutes was used, with a pad which had been soaked with 20 x SSC. After the transfer was terminated, fixation of the DNA was performed by UV irradiation.

Hybridization probe employed for the Southern analysis was a DNA probe for a portion of ORF of the cDNA sequence of the novel collectin obtained in Example 4: gaagacaagt cttcacatct tgttttcata aacactagag aggaacagca atggataaaa aaacagatgg tagggagaga gagccactgg atcggcctca cagactcaga g (SEQ ID NO: 21), which was labeled using the above-mentioned PCR DIG Probe Synthesis Kit with primers:
5'-gaagacaagtcttcaactcttg-3' (SEQ ID NO: 19) and
5'-ctctgagtctgtgaggccgatc-3' (SEQ ID NO: 20). Prior to hybridization, the probe was boiled for 10 minutes, and rapidly frozen with dry ice/ethanol for 5 minutes.

First, the membrane that was subjected to the transfer step was immersed in 2 x SSC for 5 minutes, then prehybridization was performed in ExpressHyb Hybridization Solution (Clonetech), 10 ml at 65°C for 30 minutes. Subsequently, the above frozen probe was diluted to 10 ng/ml in ExpressHyb Hybridization Solution, and 2 ml of this solution was used for hybridization at 65°C for one hour.

Following hybridization, the membrane was washed by: shaking in 20 ml of 2 x SSC solution at room temperature for 5 minutes two times, then in 20 ml of 0.2 x SSC, 0.1% SDS solution at 65°C for 15 minutes twice. Next, the membrane was washed two times with 50 ml of DIG buffer I (100 mM Tris-HCl, 150 mM NaCl (pH 7.5)) at room temperature for one minute in order to remove SDS, and then blocked in 50 ml of DIG buffer II' (1.5% blocking agent in DIG buffer 1) at room temperature for one hour. Thereafter, the membrane was treated with 10 ml of alkaline phosphatase labeled anti-DIG antibody which had been diluted to 5000-fold in DIG buffer I containing 0.2% Tween20 for 30 minutes followed by washing two times by shaking in 50 ml of DIG buffer I which contains 0.2% Tween20 at room temperature for 20 minutes. After soaking the membrane twice in 10 ml of DIG buffer III at room temperature for 3 minutes, it was placed in a hybridization bag, and CSPD (registered trade name, Boehringer Mannheim: chemiluminescence substrate) that had been diluted to 100-fold in DIG buffer III was added thereto so that the solution can spread over the membrane. Subsequently, the membrane was exposed onto Instant Film T612 (Polaroid).

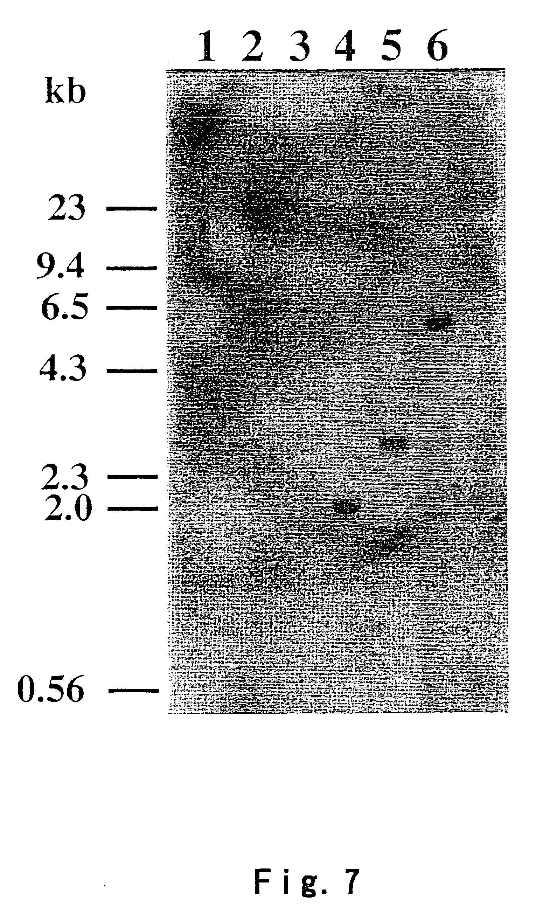

Consequently, it was speculated that the gene of the obtained novel collectin has been a single copy gene, because only one or two signals could be detected from the respective genomic DNA which was digested with each of the restriction enzymes, as shown in the lanes of Figure 7.

### Example 6: Analysis of Distribution of Human Tissue Specific Expression of the Novel Collectin

In order to examine the expression of the mRNA of the novel collectin of the present invention in various human tissues, analysis was performed by RT-PCR.

RT-PCR was performed using RNA LA PCR Kit (AMV) Ver.1.1 (TAKARA Syuzo, Co.) with each RNA derived from several human tissues ((1) brain, (2) heart, (3) kidney, (4) spleen, (5) liver, (6) small intestine, (7) muscle, (8) testis, (9) placenta, or (10) colon (OriGene Technologies, Inc.)) as a template. First, reverse transcription reaction was conducted in the following reaction mixture. The reaction mixture contained 5 mM MgCl₂, 1 x RNA PCR Buffer, 1 mM dNTP Mixture, 1 U/µl Rnase inhibitor, 0.25 U/µl reverse transcriptase, 0.125 µM Oligo dT-Adaptor Primer, RNA 1 µg, and was adjusted to total volume of 20 µl with RNase free distilled water. At the same time, a reaction mixture without reverse transcriptase was also prepared for the negative control. The reaction mixture was placed in 0.2 ml tube, and subjected to PCR with TaKaRa PCR Thermal Cycler PERSONAL (TAKARA Syuzo, Co.) through 1 cycle of: 30 minutes at 42°C, 5 minutes at 99°C, and 5 minutes at 5°C. Thus resulted PCR product was subsequently used for LA PCR with the following reaction mixture; 2.5 mM MgCl₂, 1 x LA PCR Buffer II (Mg²⁺ free), 2U TaKaRa LA Taq, added with two kinds of 0.2 µM primers (RT-PCR Primer U: 5'-gtgcccctggccctgcagaatg-3' (SEQ ID NO: 22) and RT-PCR Primer R: 5'-gcatatcaccctggggaacattttag-3' (SEQ ID NO: 23) that could amplify a cDNA sequence spanning from neck region to carbohydrate recognition domain of the novel collectin, which was adjusted to total volume of 80 µl with sterile distilled water. The reaction product was separated on 1% agarose gel electrophoresis, followed by staining with ethidium bromide solution (0.1 µg/ml), verification of the electrophoretic pattern with transilluminator, and identification of the expressed tissue.

Further, in order to compare the expressed amount in each of the tissues, RT-PCR was performed to amplify a part of β-actin with each of the tissues, and the amount of RNA was corrected. The RT-PCR was performed similarly to the above procedure with reverse transcriptase reaction, PCR reaction, and identified using 1% agarose gel electrophoresis as described above. The reaction mixture of the reverse transcription contained 5 mM MgCl₂, 1 x RNA PCR Buffer, 1 mM dNTP Mixture, 1U/µl RNase inhibitor, 0.25 U /µl reverse transcriptase, 2.5 µM random 9 mer, RNA 10 ng, which was adjusted to total volume of 60 µl with RNase free distilled water. PCR was performed through 1 cycle of: 10 minutes at 30°C, 15 minutes at 42°C, 5 minutes at 99°C and 5 minutes at 5°C. Thus resulted PCR product was subsequently used for PCR with the following reaction mixture; 1 x LA PCR Buffer II (Mg²⁺ free), 2U TaKaRa LA Taq, 0.25 µM human β-actin sense primer 5'-caagagatggccacggctgct-3'(SEQ ID NO: 24), 0.25 µM human β-actin antisense primer 5'-tccttctgcatcctgtcggca-3'(SEQ ID NO: 25), which was adjusted to total volume of 40 µl with sterile distilled water. PCR was performed through 30 cycles of: 15 seconds at 94°C, and 30 seconds at 68°C.

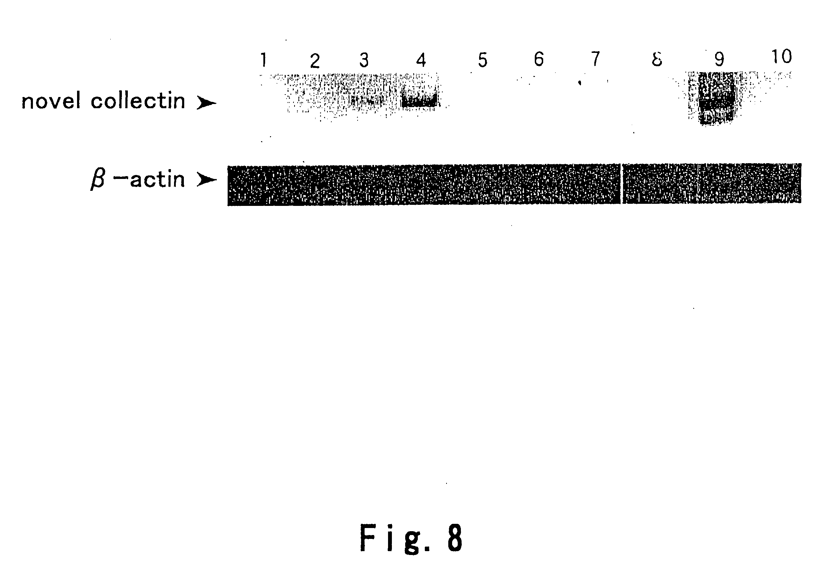

The results are shown in Figure 8, suggesting that mRNA of the novel collectin of the present invention has been expressed in placenta (lane 9), spleen (lane 4), and kidney (lane 3). Extremely high expression in placenta is clearly suggested.

### Example 7: Genomic Southern Analysis of the Novel Collectin

### from Various Animals

In order to elucidate conservation of the collectin gene of the present invention in the other species of animals, analysis by genomic Southern hybridization was performed.

Hybridization probe employed for this analysis was the DIG labeled DNA probe corresponding to the cDNA sequence of ORF of the novel collectin as described above, which was labeled using the above-described PCR DIG Probe Synthesis Kit (Boehringer Mannheim). The employed membrane was prepared by treatment of each 5 µg of genomic DNAs of (1) human (Promega), (2) monkey (Clonetech), (3) rat (Promega), (4) mouse (Promega), (5) dog (Clonetech), (6) cow (Promega), (7) rabbit (Clonetech), and (8) chicken (Promega) with restriction enzyme EcoRI, electrophoresis on agarose gel, transfer to Nytran 13N membrane, and fixation by UV irradiation.

Using the probe and membrane, hybridization was carried out according to the following procedures. First, the membrane was immersed in 2 x SSC for 5 minutes, then prehybridization was performed in 10 ml of ExpressHyb Hybridization Solution at 65°C for 30 minutes. Subsequently, the probe that had been frozen as described above was diluted in the ExpressHyb Hybridization Solution to be 10 ng/ml, and 2 ml of thus diluted probe solution was used for hybridization at 65°C for one hour.

Following hybridization, the membrane was washed by: shaking in 20 ml of 2 x SSC, 0.1% SDS at room temperature for 5 minutes two times, and then shaking in 20 ml of 0.2 x SSC, 0.1% SDS at 68°C for 15 minutes two times. Next, the membrane was washed two times with DIG buffer I at room temperature for one minute in order to remove SDS, and was blocked in 50 ml of DIG buffer II' at room temperature for one hour. Thereafter, the membrane was treated with 10 ml of alkaline phosphatase labeled anti-DIG antibody which had been diluted to 5000-fold in DIG buffer I which contains 0.2% Tween20 for 30 minutes followed by washing two times with shaking in 50 ml of DIG buffer I which contains 0.2% Tween20 at room temperature for 20 minutes. After soaking the membrane twice in 10 ml of DIG buffer III at room temperature for 3 minutes, it was placed in a hybridization bag, and CSPD that was diluted to 100-fold in DIG buffer III was added thereto so that the solution can spread over the membrane. Subsequently, the membrane was exposed to Instant Film T612.

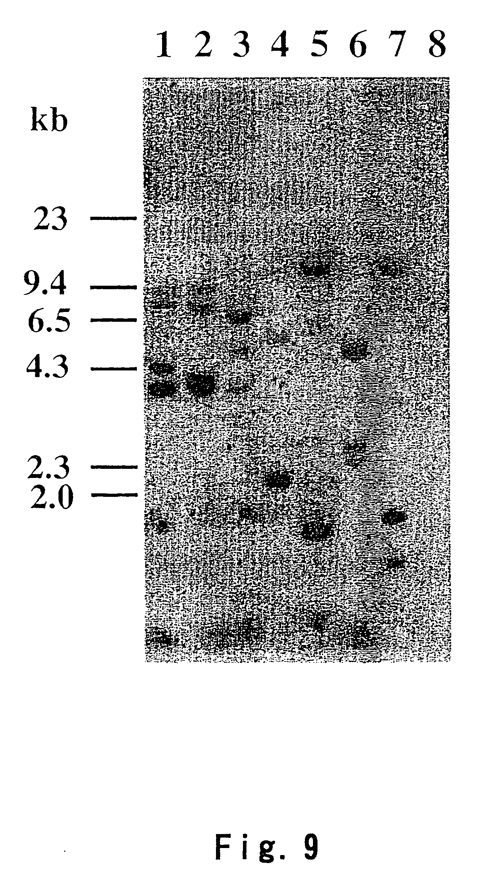

The result of this analysis is shown in Figure 9, wherein signals of DNA can been found in all lanes except for the lane 8 which was loaded with DNA from chicken. Therefore, it was demonstrated that the novel collectin gene of the present invention has been conserved between the mammalian species.

### Example 8: Genetic Analysis of the Novel Collectin

To elucidate the genetic positional relevance of the present collectin between the known collectins, analysis was performed based on the DNA sequence of the novel collectin as obtained, and a phylogenetic tree was created.

The collectins selected as subjects for analysis were several kinds of proteins belonging to the collectin family shown in Figure 10 (in Figure 10, CL-L1 designates a collectin derived from human liver, which was recently isolated by the present inventor (see, Japanese Patent Application No. Hei 10-11281)). Each of the amino acid sequence was retrieved from GenBank database, and then, multiple alignments were produced by clustalw method using the regions containing lectin domains from the obtained data to create the phylogenetic tree using N-J (neighbor-joining) method with Phylip Version 3.57c package program.

Consequently, as shown in Figure 10, although SP-D, bovine collectin-43 and bovine conglutinin have constituted single cluster, additionally MBP and SP-A have respectively constituted separate clusters, while the collectin gene of the present invention has not belonged to any of these clusters similarly to CL-L1. Furthermore, it was speculated that the collectin of the present invention may constitute a distinct cluster which is genetically diverse from those of the collectins reported heretofore including CL-L1.

### Example 9: Generation of Monoclonal Antibodies to the Novel Collectin

A solution of 100 µg of the novel collectin dissolved in 100 µl of PBS (-) (PBS, free from calcium ion and magnesium ion), and 100 µl of Freund's complete adjuvant are mixed to prepare an emulsion, and the emulsion is intraperitoneally injected into two BALB/c mice (male, 8 weeks). After three weeks, the second immunization is similarly conducted. However, Freund's incomplete adjuvant is employed instead of Freund's complete adjuvant this time. After additional two weeks, serum is recovered and the antibody titer thereof is measured. A mouse having the highest antibody titer is selected, and after one week passed, the final immunization is conducted similarly to the second immunization. After 5 days from the final immunization, the spleen is taken, and a cell suspension liquid is prepared. The cell fusion is performed by mixing 2 x 10⁷ NS-1 myeloma cells and 1 x 10⁸ spleen cells prepared. After centrifuging the mixture at 1,600 rpm for 6 minutes at room temperature, the supernatant is removed, then the remaining cells are loosen, and 1 ml of polyethylene glycol (PEG) is added thereto for a time period of one minute, followed by stirring for one minute at room temperature. Subsequently, 1 ml of culture medium (Iscove's medium, containing 15% FCS, 2 mM L-glutamine solution, 0.05 mM 2-mercaptoethanol) previously incubated at 37°C is added dropwise for a time period of one minute with gentle mixing. Following a repeat of this procedure, then a similar procedure is carried out with 8 ml of the same for a time period of 3 minutes. Subsequently, PEG is removed by centrifuging at 1,000 rpm for 5 minutes at room temperature. The pellet is loosen, to which 100 ml of 5 x 10⁶ cells/ml of thymocyte suspension liquid (for use as feeder cells enhancing hybridoma formation rate, which is suspended in hybridoma growth medium including HAT) is added, to prepare a suspension liquid. This suspension liquid is plated into a 96 well-plate, each 0.1 ml per well, then cultured at 37°C in 5% CO₂ in a CO₂ incubator.

The selection of hybridoma is carried out as follows. After one day of the initiation of the culture, 150 µl of HAT medium (hybridoma growth medium containing HAT) is added, followed by culture for the subsequent two days. After two days, the medium is replaced by removing 150 µl of the medium, and adding 150 µl of HAT medium thereto, on the following 3 and 7 days of culture, the medium is similarly replaced. After four days, 100 µl of culture supernatant is collected, then the screening is carried out through measuring the antibody titer for the novel collectin. Next, the positive cells for the antibody are transferred from the 96 well plate to a 24-well plate, using a Pasteur pipet. On this occasion, 0.5 ml of HT medium (hybridoma growth medium containing hypoxanthine and thymidine) has been previously added to each well. After culturing 3 days, a portion of the medium is collected, and its antibody titer is again measured, consequently, the cells capable of being determined as antibody-positive are cloned and frozen for storage. For cloning, the positive cells are subjected to limiting dilution in the thymocyte suspension liquid to adjust to 1 cell/ml, each of 0.2 ml is plated into a 96 well-multi plate. After culturing 10 days, the antibody titer of the well that forms single colony per well is measured, and the two colonies indicating higher antibody titers are selected. These two clones are subjected to recloning. The cloning method is carried out similarly to the method of the first cloning. Subsequently, the extensive culture of the antibody-positive cell is conducted. First, after transferring the cell to a 24 well-multi plate, the cell is cultured for 3 days, followed by transfer to a 25 cm² flask, further culturing 3 days, and again transfer to 225 cm², and culturing 3 days, accordingly, a monoclonal antibody is prepared by recovering the culture supernatant.

Moreover, for generating a monoclonal antibody from ascites fluid, the cloned hybridoma is adjusted to 1 x 10⁷ cells/ml, and then I ml per mouse is injected intraperitoneally. After one week, the mouse with enlarged abdomen is anesthetized with ether, and the ascites fluid is collected. The ascites fluid is transferred to a coagulation promoting type centrifugation tube added with serum separation agent, followed by allowing to stand for 30 minutes at room temperature, and centrifugation at 3,000 rpm for 5 minutes. Ascites fluid from upper portion is recovered, and then the fluid is further purified by ammonium sulfate fractionation to obtain an antibody.

### Example 10: ELISA Method Using Anti-mouse Anti-human Novel Collectin Antibody

Anti-mouse anti-human novel collectin antibody is diluted in coating buffer (50 mM carbonate - bicarbonate buffer, pH 9.6) to 10 µg/ml, and the antibody liquid is dispensed 100 µl per well in a 96-well ELISA plate. Subsequently, the plate is incubated at 4°C overnight. The wells were washed three times with 300 µl of a wash liquid (TBS/T liquid (25 mM Tris-HCl, 0.14 M NaCl, 5 mM KCl, 0.05% Tween 20, pH 7.4)). Hereinafter, washing may be similarly performed. Three hundred µl of 5% skim milk/TBS/T liquid is respectively dispensed to the wells, and allowed to stand at 37°C for one hour. After washing, 100 µl of a test sample and a novel collectin standard are dispensed thereto, and the plate is allowed to stand at 37°C for one hour. On this occasion, the test sample is properly diluted, and two-fold serial dilution is employed up to the highest concentration of 0.02 µg/ml for the standard. After washing, 100 µl of 1 µg/ml biotinized anti-rabbit ant-human novel collectin antibody is dispensed thereto, and the plate is allowed to stand at 37°C for 30 minutes. After further washes, 100 µl of biotin streptavidin solution is dispensed thereto, and the plate is allowed to stand at 37°C for 30 minutes. After additional washes, 100 µl of 3, 3', 5, 5'-tetramethylbenzidine solution is dispensed, and the plate is allowed to stand for 30 minutes at room temperature, followed by addition of 1N phosphoric acid 100 µl, termination of the reaction, and measurement of the absorbance at 450 nm of the wavelength.

A calibration curve with the standard is produced using this method, and the quantitative determination of the novel collectin is accomplished.

### Example 11: Generation of Antibodies with Lowered Immunogenicity to Human

First, mRNA is prepared from the mouse hybridoma obtained in the method described in Example 9, a cDNA library with a phage vector is prepared, and then cDNA cloning is performed. A mouse antibody cDNA is cloned using cDNA of a constant region as a probe, followed by analysis of the inserted cDNA with restriction enzymes, and then the nucleotide sequence of the antibody cDNA is sequenced.

Next, a chimeric antibody is expressed, and the presence or absence of a mutation in the cloned antibody gene is confirmed from the antibody binding activity, while it may be used as a positive control for studying the binding activity of the expressed humanized antibody. When the chimeric antibody is expressed, a cassette type expression vector in which a constant region gene of human antibody H chain and L chain is cloned on single vector may be employed. Binding activity of the chimeric antibody is determined, resulting in confirmation that the gene is undoubtedly the cloned mouse antibody gene, then a humanized antibody gene is constructed. In the case of preparing a humanized antibody, a gene must be designed for construction, as is different from the case of chimeric antibody generation method. On the basis of the determined nucleotide sequence of the mouse antibody variable region gene, an amino acid sequence of a human antibody variable region that is highly homologous is searched by utilizing a database. The sequences having 70-80% of homologies other than CDR sequence are found, thereafter a mouse antibody CDR sequence is grafted to the region in which CDR sequence is removed from thus selected human antibody variable region (framework region), accordingly, molecular modeling is carried out by a protein structure analysis software. A few amino acids of the respective H chain and L chain in a sequence of the human framework region are returned back to those of the mouse type so that the configurations of 6 CDRs in total on the H chain and L chain that bind to an antigen through modeling become closer to those of the original mouse monoclonal antibody in respect to the three dimensional structure. In such a case, returning the less number of the amino acids to the mouse type is more desirable, because the closer antibody to the human antibody can be resulted. According to this molecule modeling, more than 10 kinds of versions of humanized antibody genes are designed, expressed and activities thereof should be compared, because the probability that the humanized antibody started from one model can reproduce an activity of 100%, equivalent to that of the mouse monoclonal antibody would be low.

After the gene design is completed, the construction of the gene is actually performed. A cassette type expression vector including a constant region gene of a humanized antibody H, L chains is cloned, then a transient expression is carried out with COS cells and the like, thus the antibody activity of the culture medium supernatant is determined by ELISA method. Several kinds having strong activities are selected, and the stable expression cells are established by employing the respective expression vectors for stable expression and host cells such as COS cells, to prepare the purified antibody from the culture medium supernatant.

### Example 12: Construction of an Expression Vector pNOW1-hCL-P1 of the Novel Collectin

First, a sequence spanning from the initiation codon to termination codon of the novel collectins set out in SEQ ID NO: 1 is amplified using a primer consisted of the nucleotide sequence: aaggaaaaaa gcggccgcat gcaacaagat ttgatgagg (SEQ ID NO: 26), and a primer consisted of the nucleotide sequence gctctagatt ataatgcaga tgacagtac (SEQ ID NO: 27) with Zymoreactor (ATTO Corp.,).

The cDNA of the novel collectin obtained is digested with restriction enzymes NotI and XbaI, yielding a cDNA portion corresponding to 670-1698 bp of cDNA of the novel collectin, which may be an insert.

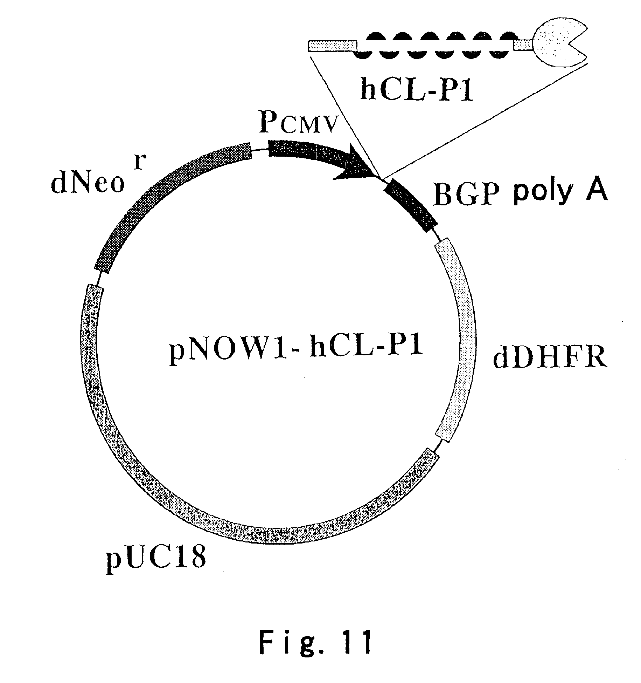

Next, the expression vector pNOW/CMV-A (see, Japanese Unexamined Patent Publication No. H10-179169 (1998)) is digested with restriction enzymes NotI and XbaI, then the above insert is inserted at downstream of cytomegalovirus promoter (pCMV), namely, between P_{CMV} (cytomegalovirus major antigen early promoter) and BGP poly A (bovine growth hormone polyadenylation signal), using a DNA ligation kit (Takara Shuzo Co., Ltd.). The expression vector thus obtained is designated as plasmid pNOW1-hCL-P1 (hCL-P1: a nucleotide sequence encoding the novel collectin), and a schematic drawing of its structure is shown in Figure 11.

### Example 13: Selection of a Clone Expressing the Novel Collectin

### (1) Introduction of the expression vector pNOW1-hCL-P1 into dihydrofolate reductase deficient Chinese hamster ovary (CHO) cells (dhfr⁻)

Iscove's Modified Dulbecco's Medium (IMDM, GIBCO) that is added with 10% of fetal calf serum (FCS, GIBCO) (free from hypoxanthine and thymidine) is prepared, to which DHFR gene deficient DG44 CHO cell strain (dhfr⁻) is mixed to yield 1 x 10⁵ cells/ml, which is seeded into a dish having 60 mm diameter, and cultured at 37°C for 24 hours under a condition of 5% carbon dioxide (CO₂). The culture medium supernatant is discarded, and in turn, IMOM containing 100 µl solution separately and previously obtained by mixing 5 µg of DNA (expression vector pNOW1-hCL-P1) with lipofectin solution (DOTAP: Liposomal Transfection Reagent; Boeringer Mannheim) with 10% FCS is added to be 6 ml, further, hypoxanthine (final concentration of 10 nM) (GIBCO) and thymidine (final concentration of 100 nM) (GIBCO) are added thereto, followed by culturing for 16 hours, to introduce the expression vector pNOW1-hCL-P1 into the host dhfr⁻ CHO cells of. Thereafter, the culture medium supernatant is discarded, followed by addition of 6 ml of IMDM added with 10% FCS, hypoxanthine, and thymidine, and further culture for 24 hours.

### (2) Acquisition of neomycin (G418) resistant CHO cells

After the cells in which the expression vector pNOW1-hCL-P1 is incorporated are cultured for 24 hours, which are trypsinized and recovered from the dish, the number of the cells are counted, and thereafter the mixture in which those cells are suspended in IMDM containing neomycin (G418) at a concentration of 400 µg/ml with 10% FCS is seeded (dispensed) into ten 96-well micro plates at an amount of 0.1 ml/well. The plates are cultured at 37°C under a condition of 5% carbon dioxide (CO₂), and after two weeks, the surviving cells are acquired as G418 resistant cells (clone).

These G418 resistant clones are identified for hCL-P1 producibility. Several clones are selected among the clones in which the production of hCL-P1 is confirmed, and then each of the selected clones is seeded into a culture flask of 25 cm². The culture is carried out until the cells become confluent (approximately 3 x 10⁶ cells/25 cm² culture flask). The culture medium supernatant from each culture flask is discarded, then 2 ml of the IMDM containing 10% FCS identical to the composition described above is added thereto, followed by culture for four days, and recovering the culture medium supernatant. The produced amount of hCL-P1 (rhCL-P1: recombinant human novel collectin) in the recovered culture medium supernatant is measured. It should be noted that the produced amount of hCLP1 may be quantitatively determined according to the method of Suzuki *et al.* (Y. Suzuki, *et al.,* "Characterization of Recombinant Bovine Conglutinin Expressed in a Mammalian Cell", *Biochem. Biophys. Res. Commun.,* **238**:856-863 (1997)) using an anti-rabbit polyclonal antibody to hCL-P1, carbohydrate recognition domain (CRD) and neck region of collectins (expressed in E. coli using a similar method), that is expressed in *E. coli* using the method of Suzuki, *et al., supra,* as well as hCL-P1 (quantitative reference) as a control.

### (3) Acquisition of methotrexate resistant CHO cells

Following further passage culture and stabilization of the hCL-P1 producing clone obtained in the above-described Example 13 (2), low concentration of methotrexate (MTX) is added to the medium, and gene amplification is effected.

First, the selected each cell clone is mixed in IMDM containing 5 nM MTX, 400 µg/ml of G418 with 10% FCS previously dialyzed (JRH Bioscience), is seeded (dispensed) into ten 96-well micro plates at 0.1 ml/well. The cells are cultured at 37°C under a condition of 5% carbon dioxide (CO₂), and after two weeks, the surviving cells are acquired as 5 nM MTX resistant cells (clone).

These 5 nM MTX resistant clones are identified for hCL-P1 producibility. Several clones are optionally selected among the clones in which the production of hCL-P1 is confirmed, and then each of the selected clones is seeded into a culture flask of 25 cm² followed by two weeks culture until the cells become confluent. The culture medium supernatant is discarded, then 2 ml of the IMDM containing 10% FCS identical to the composition described above is added thereto, followed by culture for four days, recovering the culture medium supernatant, and then the production level of hCL-P1 is measured. It should be noted that the quantitative determination of the produced amount of hCLP1 may be performed according to the ELISA method described in Example 10.

### Example 14: Production of a Transgenic Mouse

Collectins are believed to have important roles in basic immunity. Additionally, since the novel collectin of the present invention is conceived to form a genetically independent cluster from the conventional collectins, a transgenic non-human animal of this novel collectin is produced in order to obtain new knowledge on immunity and to provide important tools for elucidating the mechanisms of action of the novel collectin.

A plasmid is constructed, which is capable of expressing the novel collectin derived from human of the present invention in a mouse. First, a 2.3 kb fragment is excised by digestion with Sal I/Pst I from a mammalian animal expression vector comprising a chicken β-actin promoter and a cytomegalovirus enhancer at upstream of the β-actin promoter (Niwa, H. *et al., Gene,* **108:**193-200, 1991), which is inserted into Sal I/Pst I site of a cloning vector pBluescript (Stratagene), resulting in construction of pBsCAG-2 vector. In this 2.3 kb fragment, the above described enhancer/ promoter and a portion of rabbit β-globin gene (the second intron, the third exon, and 3'-noncoding region) are contained. The cDNA of hCL-P1 (SEQ ID NO: 1, 670-1698) is inserted into an Eco RI site of the above- described third exon, and a plasmid pβA/hCL-P1 for use in expression in a transgenic mouse is constructed.

Next, a large amount of plasmid pβA/hCL-P1 is prepared by an alkaline-SDS method, and the plasmid is purified by CsCl equilibrium density-gradient ultracentrifugation method. A gene is isolated by digestion of pβA/hCL-P1 with Sal I/Bam HI, thus the portion of the vector is excised. Subsequently, the gene is separated from the vector DNA fragment by agarose gel electrophoresis, and the introduced DNA fragment is excised from the gel, followed by purification of the DNA fragment using GeneClean II Kit. After ethanol precipitation, the DNA fragment is dissolved in DNA solution for injection (10 mM Tris-HCl (pH 7.5), 0.25 mM EDTA), followed by centrifugation at 15,000 rpm for 5 minutes and the supernatant is recovered. DNA concentration is calculated through measuring absorbance using spectrophotometer, and after adjusting the concentration to 500 DNA molecules/p1, the solution is dispensed to 50 ml, and stored at -20°C until the DNA is introduced into the first phase embryo of a mouse.

Next, fertilized mouse eggs for injecting DNA is prepared. First, in order to induce superovulation in female mouse for use of collecting fertilized eggs, 5 IU/body of pregnant horse serum gonadotropin (PMSG) and human chorionic gonadotropin (hCG) are administered intraperitoneally at intervals of 48 hours, and the female mouse is subjected to cohabitation with a male mouse. Abdominal cavity of the female mouse is opened, of which vaginal plug is formed, then the uterine tube is eviscerate, washed with BWW-Hepes medium (containing NaCl 102.2 mM, KCl 4.78 mM, KH₂PO₄ 1.19 mM, CaCl₂ 1.17 mM, MgSO₄ 1.19 mM, NaHCO₃ 4.76 mM, Hepes 15 mM, sodium lactate 25 mM, sodium pyruvate 0.25 mM, glucose 5.56 mM, BSA 4 mg/mL, EDTA 0.1 mM and phenol red 0.00025%), thereafter, placed in BWW-Hepes medium including 300 mg/ml of hyaluronidase. Ampulla of the uterine tube is cleaved by a watch marker's tweezer under a stereoscopic microscope, then the fertilized egg is dislodged into the medium, picked up by a glass pipet, and washed five times in BWW-Hepes medium. Thereafter, the fertilized egg is placed into a drop of BWW medium covered by mineral oil, and cultured at 37°C under a condition of 5% CO₂ in an incubator.

Subsequently, the DNA is injected into the fertilized egg pronucleus. In a 60 mm petri dish, a drop of BWW-Hepes medium and DNA solution (500 DNA molecules/pl) is generated, covered with mineral oil. Using injectors respectively equipped with an injection pipet and a holding pipet, the fertilized egg is entered into the drop over the BWW-Hepes medium. The DNA solution is aspirated in the injection pipet, and the injector is adjusted to be slightly positive pressure, so that the DNA solution is allowed to flow continuously from the tip. The fertilized egg is sucked by the holding pipet so that the male pronucleus is positioned to the injection pipet side, and then the magnification of the objective lens is changed to 40 fold followed by focusing to the pronucleus, the position of the tip of the injection pipet is adjusted to the same plane with the pronucleus.

Subsequently, the tip of the injection pipet is penetrated into the pronucleus, and the DNA solution is injected. After swelling of the nucleus is confirmed, the pipet is quickly drawn, then the injected egg is moved to the lower drop, and manipulated so that mixing with the untreated eggs is avoided. About 30 eggs are subjected to injection once, subsequently, moved to a drop of the BWW medium, and cultured at 37°C under a condition of 5% CO₂ in an incubator.

After culturing, the transplantation of the fertilized eggs to uterine tube is carried out. Nembutal injection liquid is diluted to 10-fold by a diluent (to a final concentration of 5 mg/ml), and 0.01 ml of the solution is administered intraperitoneally per 1 kg of weight, to anesthetize a pseudopregnant mouse. About 30 eggs are injected into a uterine opening of the uterine tube, using a transfer pipet which has been previously sucked the eggs thereinto along with the medium as little as possible (0.5-1.0 ml). The ovary and uterine tube are returned within the body, and the opening is sutured. Subsequently, the breeding is continued, and approximately 19 days later, an infant is born.

Screening of the transgenic mouse is carried out by dot blot hybridization as follows. The obtained infant is weaned at four weeks after birth, of which tail is cut at about 1 cm from the top under anesthesia, and the piece is placed in an Eppendorf tube of 1.5 ml. STE buffer (50 mM Tris-HCl, 100 mM EDTA (pH 7.5), 0.5% (w/v) SDS) containing 0.5 mg/ml of proitenase K is added at 0.5 ml to the tube, which is subjected to shaking at 55°C in an incubator overnight. One hundred µl of the tail portion solution is transferred to a new tube, then TE saturated phenol 200 µl and TE buffer 110 µl are added thereto, followed by subjecting to the vortex mixer for 5 minutes, and the mixture was centrifuged at 15,000 rpm (20,000 x g) for 5 minutes at room temperature. Two hundred µl of thus resulted aqueous layer is transferred to a new tube, and 200 µl of phenol/chloroform is added thereto, followed by subjecting to the vortex mixer for 5 minutes, and the mixture is centrifuged at 15,000 rpm (20,000 x g) for 5 minutes at room temperature. The supernatant is transferred to a new tube, 20 µl of 3M sodium acetate and 500 µl of ethanol are added thereto, then the mixture is sufficiently shaken for mixing until the filamentous precipitation of DNA can be visually observed, thereafter, subjected to centrifugation at 15,000 rpm (20,000 x g) at 4°C for 5 minutes. After removing the supernatant followed by washes with 70% ethanol, and drying for 5 minutes, the DNA precipitate is dissolved in 100 µl of 2M NaCl, 0.1 M NaOH solution. Following heating for 5 minutes in a boiling water bath, the DNA is rapidly quenched in ice-cold water, thus denaturation of the DNA is resulted. After a 96-well manifold is set, the DNA is spotted on a nylon membrane, then the membrane is air-dried, and heated at 80°C for 2 hours. Hybridization is performed by a conventional method using as a probe a portion without a sequence derived from mouse among the introduced DNA. As a result, the individual in which the signal can be confirmed is identified as transgenic mouse.

When the manipulation of gene injection is thus performed into the fertilized eggs, about 90% of the fertilized eggs keep surviving. All of these surviving eggs are transplanted into recipients at approximately 25 eggs per one animal so that the recipients become pregnant, and about 19th day of the pregnancy, the born infant can be obtained. Genetic analysis of these infant mice is performed, and the existence of the introduced gene is confirmed. Furthermore, F1 mouse is obtained from these transgenic mice, and transmission rate of the transgene is also examined.

### Example 15: Production of a knockout mouse

First, the construction of DNA for targeting is conduced by incorporating a neomycin resistant gene, which is a selectable marker gene, into an exon portion of a part of the objective gene coding a mouse homologue of a novel collectin of the present invention in ES cell derived from C57BL/ 6J strain mouse. Next, electroporation of the DNA to ES cells is carried out as follows. The medium of ES cells at a logarithmic growth phase (one 90 mm petri dish) is changed to a fresh ES medium (80% DMEM medium containing 20% FCS, 1 mM pyruvic acid solution, 0.1 mM non-essential amino acid solution and 1 mM 2-mercaptoethanol), followed by additional culture for 4 hours, then after removing the medium and sufficient washes with PBS (-), the cells are treated with 0.1 % trypsin for 3 minutes. The cells are added with 1 ml of the medium, then dispersed to single cells, centrifuged at 600 rpm at 4°C for 5 minutes, then the after discarding the supernatant, the cells are suspended in 5 ml of PBS (-), and the number of the cells are counted. Again, the cells are centrifuged at 600 rpm at 4°C for 5 minutes, and the supernatant is discarded, thereafter the cells are suspended in PBS (-) to be 1.1 x 10⁷ cells/ml. From the suspension of 1.1 x 10⁷ cells/ml, 0.9 ml is transferred to a cuvette, which is added with 1 µg/µl of DNA solution (for targeting) 25 µl (25 µg), and allowed to stand at room temperature for 5 minutes. The electroporation is carried out using a gene pulser under a condition of 50C µF and 230 V, followed by standing for 5 minutes, the cells are suspended in ES medium (-G418), and seeded each 1/4 portion on four petri dishes (90 mm) that has been plated with feeder cells. After the electroporation is completed, followed by culture for 24 hours, the medium is changed to ES medium containing 150 µg/ml G418 (active type), and the medium is changed on the following everyday. Seven days later, the cloning is carried out as follows.

The medium is removed from the 90 mm petri dish in which the colony appeared, and 10 ml of PBS (-) is dispensed. Colonies in good shapes observed under a stereoscopic microscope are scraped with a tip respectively, sucked, and each of the colonies is transferred to a 96-well plate in which 70 µl of 0.1% trypsin solution has been previously placed. The plate is allowed to stand at 37°C for 4-5 minutes as it is, followed by trypsinization, and after pipetting is sufficiently conducted using a 8 Channel Pipetman, the cells are seeded into four wells of a 24-well plate (with feeder cells adsorbed) in which 0.8 ml of ES medium containing 75 µg/ml G418 has been previously placed. After the culture for 24 hours, the medium is changed to ES medium containing 75 µg/ml G418. Three days later, 100 µl of 0.1% trypsin is added to the clone at a logarithmic growth phase, and after 100 µl of ES medium is added thereto followed by pipetting, the clone is transferred to a round bottom 96-well plate. When all of the 48 clones are treated, 20 µl is taken for culture, while 180 µl of the remainder is used for PCR.

After the clone for PCR in the 96-well plate is centrifuged at 1,200 rpm, at 4°C for 5 minutes, the medium is removed, and the cells are washed three times with PBS (-). One hundred µl of the dissolving solution (100 mM Tris-HCl (pH 8.0), 10 mM EDTA, 0.2% (w/v) SDS, 100 mM NaCl, 100 µg/ml proitenase K) is added to the clone using a 8 Channel Pipetman, thus proteinase K-treatment is carried out at 55°C for 1 hour. The plate is sealed with a tape adhered around the plate, and further incubated at 95°C for 15 minutes for complete inactivation of proitenase K. The plate is cooled at 4°C for 5 minutes, 2-3 µl of the solution is collected as a sample for PCR, and used for the first screening (the remaining solution is stored at -20°C). The sample may be a group consisting of 4 clones, and PCR is performed using a sense primer (atgcaacaagatttgatgagg (SEQ ID NO: 28) and an antisense primer (cctacccggtagaattgacc (SEQ ID NO: 29), followed by identification of the positive groups through determination of the objective gene by the electrophoresis method. Among the remaining samples that have been conserved at -20°C, each clone belonging to the positive groups are similarly subjected to PCR, and the positive clones are identified. Next, the screening for clones for use in culture is performed. Twenty µl of the clone for the culture is seeded into four wells/line of a 24-well plate (with feeder cells adsorbed), and when all wells are filled, the plate is placed in a CO₂ incubator and cultured, then at a final stage, one clone is divided into three wells and successively seeded into a 6 wells-plate (with feeder cells adsorbed).

Generation of a germ line chimera mouse is performed by a sandwich method where an ES cell mass in which a gene is introduced is sandwiched between two pieces of 2.5 days old embryos (8 cells-phase embryo) of a mouse, which are prepared as described below.

The preparation of 2.5 days old embryo is conducted as follows. Five IU of PMSG (pregnant mare serum gonadotropin) is administered intraperitoneally into a C57BL 6J strain female mouse for use of collecting eggs, on 6 days before the adhesion with the 2.5 days ES cells is conducted. On 4 days therebefore, 5 IU of hCG (human chorionic gonadotropin) is additionally administered intraperitoneally, and the mouse is lead to mating with a male. On 3 days before, mating is confirmed by vaginal plug formation, and a female mouse for pseudopregnant being naturally estrous is lead to mating with a male mouse received vasoligation. The mating is confirmed by vaginal plug formation, thus the female mouse is identified as a pseudopregnant mouse. On the day of adhesion, the female mouse for collecting eggs is subjected to euthanasia by dislocation of cervical vertebrae, and then uterine tube and uterus are removed. The uterine tube and uterus are refluxed with BWW-Hepes medium, 2.5 days embryos (8 cells-phase embryos, or morula embryos) are collected, transferred to a drop of BWW medium, and cultured in a CO₂ incubator (5% CO₂, 37°C). The eggs are collected, and the 2.5 days embryos are transferred to a drop of an acidic Tyrode's solution (200 µl), allowed to stand for 1-2 minutes, thereafter, when clear zone is dissolved, the eggs are immediately washed six times with BWW-Hepes medium and is used for adhesion with the ES cells.

The preparation of ES cells is conducted as follows. On the day of adhesion, ES cells cultured on feeder cells are washed two times with PBS (-), subjected to trypsinization with a 0.05% trypsin liquid for 2 minutes, and scraped from the petri dish to yield a mass of several tens of the cells. ES medium (-G418) is added to the cells to inactivate trypsin, and after centrifugation, the cells are suspended in ES medium. They are is seeded in a petri dish covered with 0.1% gelatin, and allowed to stand for 15 minutes in a CO₂ incubator. Cells not adhered to the dish are collected, and these cells are used for preparation of an aggregated chimera.

The adhesion between the 2.5 days embryos and ES cells is performed as follows. First, wells are made on the bottom of a petri dish for bacterial culture using a needle. The well is covered with a drop of 20 µl of BWW medium, and further covered with mineral oil. Then, the dish is placed in a CO₂ incubator, thus the medium is equilibrated to the condition. The masses consisting of 10-20 ES cells having a good shape are selected under a stereoscopic microscope. Those masses of ES cells are placed into the wells prepared as above one by one. The 2.5 days embryos of which clear zones have been removed are placed into the wells at 2 embryos per well to achieve adhesion to the mass of ES cells. The dish is cultured overnight in a CO₂ incubator, and one blastocyst is resulted having an almost equivalent size to that of 2 pieces.

Next, 3.5 days embryos are transplanted into uterus. Nembutal parenteral solution is administered intraperitoneally (50 µg per 1g of body weight) to a pseudopregnant mouse (2.5 days after mating) for anesthetization. The dorsal side of the anesthetized mouse is dissected, and the uterus is extracted through clutching fat mass. The blastocysts or morulas prepared by culturing overnight are sucked up along with a small amount of medium (0.5-1.0 µl) at 10-15 pieces each to a pipet, thereafter, those embryos are transplanted into a uterine by puncturing the uterine wall at a part close to the uterine tube with a needle of syringe, and inserting a pipet through the opening followed by ejection. The uterine is returned within the body, and the opening is sutured, subsequently, approximately 18 days later, an infant is born.

After transmission to the germ line is confirmed, the mice which are heterologous each other are crossed to result in homogenization, accordingly, an objective knockout mouse that is not expressing a homologue of a novel collectin of the present invention is obtained.

### [Industrial Applicability]

As set forth above, a novel collectin and a gene encoding the novel collectin having characteristic structures of the collectins that have been demonstrated to exhibit anti-bacterial, anti-viral activities, which are different from the collectins reported so far, are provided by the present invention.

The sequence information presented by the present invention is useful for investigating mechanisms of biological defense systems, and may provide medical, experimental tools in which biological activities of the novel collectin are utilized. For example, vectors that can express the novel collectin, host cells comprising the vector with feasibility of expression, antibodies for the novel collectin, as well as probes for screening the related molecular species of the novel collectin can be provided.

In addition, transgenic non-human animals, and knockout non-human animals are described, which may be utilized as disease model animals for studies on functions, or regulation of expression of the novel collectin and/or the derived molecules, for elucidating the mechanisms involved in diseases related to the cells where those molecules are expressed in human, and for screening and safety test of pharmaceutical products.

## Claims

1. A novel human collectin consisting of the amino acid sequence of SEQ ID NO: 2, residues 206-547.

2. A novel human collectin consisting of the amino acid sequence of SEQ ID NO: 2, residues 229-547.

3. A novel collectin according to claim 2, wherein said novel collectin further consists of an amino acid sequence upstream of the first methionine residue (SEQ ID NO: 2, residue 229) selected from SEQ ID NO: 2, residues 226-228, 211-228, 102-228, 91-228, 9-228 and 1-228.

4. An isolated polynucleotide consisting of a nucleotide sequence that encodes a protein having the amino acid sequence of any one of claims 1 to 3.

5. An isolated polynucleotide consisting of a nucleotide sequence having the sequence of SEQ ID NO: 1, bases 670-1695.

6. An isolated polynucleotide consisting of a nucleotide sequence having the sequence of SEQ ID NO: 1, bases 739-1695.

7. A polynucleotide according to claim 6 further consisting of a nucleotide sequence 5' upstream of said nucleotide sequence which is selected from SEQ ID NO: 1, bases 730-738, 685-738, 358-738, 325-738, 79-738, 55-738 and 1-738.

8. A polynucleotide according to any one of claims 5 to 7 which further consists of the nucleotide sequence of SEQ ID NO: 1, bases 1696-2024 downstream of said nucleotide sequence.

9. An isolated polynucleotide encoding a novel collectin, which hybridizes with a probe that is an amplification product from a PCR performed using primers having the nucleotide sequences of SEQ ID NO: 4 and SEQ ID NO: 5 under the following hybridization conditions prehybridization in a solution of 5 x SSC, 1% blocking agent, 0.1% N-lauroyl sarcosine, and 0.02% SDS, at 68°C for an hour; hybridization in a solution of 5 x SSC, 1% blocking agent, 0.1% N-lauroyl sarcosine, and 0.02% SDS containing cDNA probes (10 ng/ml), at 55°C for 16 hours; washing in a solution of 2 x SSC/0.1% SDS for 5 minutes, 2 times; and washing in a solution of 0.5 x SSC/0.1% SDS at 55°C for 15 minutes, 2 times.

10. A polynucleotide which hybridizes with any one of the polynucleotides according to any one of claims 4 to 9 under the following hybridization conditions prehybridization in a solution of 5 x SSC, 1% blocking agent, 0.1% N-lauroyl sarcosine, and 0.02% SDS, at 68°C for an hour; hybridization in a solution of 5 x SSC, 1% blocking agent, 0.1% N-lauroyl sarcosine, and 0.02% SDS containing cDNA probes (10 ng/ml), at 55°C for 16 hours; washing in a solution of 2 x SSC/0.1% SDS for 5 minutes, 2 times; and washing in a solution of 0.5 x SSC/0.1% SDS at 55°C for 15 minutes, 2 times, wherein the protein encoded by said polynucleotide is a novel collectin which comprises: (1) a Ca²⁺-dependent carbohydrate recognition domain (CRD), and (2) a collagen-like region.

11. A polynucleotide according to any one of claims 4 to 10, wherein said polynucleotide is cDNA.

12. A novel collectin consisting of the amino acid sequence encoded by the polynucleotide according to any one of claims 5 to 11.

13. A vector which comprises the polynucleotide according to any one of claims 4 to 11 in a manner that allows expression of the novel collectin.

14. A host cell comprising the vector according to claim 13 in a manner that allows expression of the novel collectin.

15. A probe for screening a novel collectin related molecular species consisting of the polynucleotide according to any one of claims 4 to 11.

16. A transgenic non-human animal that is stably introduced with a recombinant gene comprising the polynucleotide according to any one of claims 4 to 11 into the chromosome, and that can express a novel collectin.

## Patentansprüche

1. Ein neues menschliches Collektin bestehend aus der Aminosäuresequenz von SEQ ID NO: 2, Reste 206-547.

2. Ein neues menschliches Collektin bestehend aus der Aminosäuresequenz von SEQ ID NO: 2, Reste 229-547.

3. Ein neues Collektin gemäß Anspruch 2, wobei das neue Collektin ferner aus einer Aminosäuresequenz besteht, die dem ersten Methioninrest (SEQ ID NO: 2, Rest 229) vorangehend und ausgewählt ist aus SEQ ID NO: 2, Reste 226-228, 211-228, 102-228, 91-228, 9-228 und 1-228.

4. Ein isoliertes Polynukleotid, bestehend aus einer Nukleotidsequenz, die ein Protein kodiert, das die Aminosäuresequenz einer der Ansprüche 1 bis 3 hat.

5. Ein isoliertes Polynukleotid bestehend aus einer Nukleotidsequenz, die die Sequenz SEQ ID NO: 1, Basen 670-1695 hat.

6. Ein isoliertes Polynukleotid bestehend aus einer Nukleotidsequenz, die die Sequenz SEQ ID NO: 1, Basen 739-1695 hat.

7. Ein Polynukleotid gemäß Anspruch 6, ferner bestehend aus einer Nukleotidsequenz 5' vorangehend der Nukleotidsequenz, welche ausgewählt ist aus SEQ ID NO: 1, Basen 730-738, 685-738, 358-738, 325-738, 79-738, 55-738 und 1-738.

8. Ein Polynukleotid gemäß einem der Ansprüche 5 bis 7, welches ferner der Nukleotidsequenz nachfolgend aus der Nukleotidsequenz von SEQ ID NO: 1, Basen 1696-2024 besteht.

9. Ein isoliertes Polynukleotid, welches ein neues Collektin kodiert, das mit einer Probe, die ein Verstärkungsprodukt ist, gebildet aus einem PCR unter Verwendung von Primern, die die Nukleotidsequenzen von SEQ ID NO: 4 und SEQ ID NO: 5 haben, unter den folgenden Hybridisierungsbedingungen hybridisiert, Vorhybridisierung in einer Lösung von 5 x SSC, 1 % Blockmittel, 0,1 % N-Lauroyl Sarcosin, und 0,02 % SDS, bei 68°C für eine Stunde; Hybridisierung in einer Lösung von 5 x SSC, 1 % Blockmittel, 0,1 % N-Lauroyl Sarcosin, und 0,02 % SDS enthaltend cDNA Proben (10 ng/ml), bei 55°C für 16 Stunden; Waschen in einer Lösung von 2 x SSC/0,1 % SDS für 5 Minuten, 2mal; und Waschen in einer Lösung von 0,5 x SSC/0,1 % SDS bei 55° C für 15 Minuten, 2mal.

10. Ein Polynukleotid, welches mit einem der Polynukleotide gemäß einem der Ansprüche 4 bis 9 unter den folgenden Hybridisierungsbedingungen hybridisiert, Vorhybridisierung in einer Lösung von 5 x SSC, 1 % Blockmittel, 0,1 % N-Lauroyl Sarcosin und 0,02 % SDS, bei 68° für eine Stunde; Hybridisierung in einer Lösung von 5 x SSC, 1 % Blockmittel, 0,1 % N-Lauroyl Sarcosin und 0,02 % SDS enthaltend cDNA Proben (10 ng/ml), bei 55° C für 16 Stunden; Waschen in einer Lösung von 2 x SSC/0,1 % SDS für 5 Minuten, 2mal; und Waschen in einer Lösung von 0,5 x SSC/0,1 % SDS bei 55° C für 15 Minuten, 2mal, wobei das Protein, das durch das Polynukleotid kodiert ist, ein neues Collektin ist, welches umfasst: (1) eine CA²⁺-abhängige Kohlenhydrat-Wiedererkennungsdomäne (CRD), und (2) eine Collagen ähnliche Region.

11. Ein Polynukleotid nach einem der Ansprüche 4 bis 10, wobei das Polynukleotid cDNA ist.

12. Ein neues Collektin bestehend aus der Aminosäuresequenz, die durch das Polynukleotid gemäß einem der Ansprüche 5 bis 11 kodiert ist.

13. Ein Vektor, welcher das Polynukleotid gemäß einem der Ansprüche 4 bis 11 in einer Weise enthält, die Ausprägung des neuen Collektins erlaubt.

14. Eine Gastzelle, die den Vektor nach Anspruch 13 in einer Weise enthält, die Ausprägung des neuen Collektins erlaubt.

15. Eine Probe zum Screenen einer auf ein neues Collektin bezogenen Molekülspezies bestehend aus dem Polynukleotid gemäß einem der Ansprüche 4 bis 11.

16. Ein transgenes nicht menschliches Tier, in welches dauerhaft ein rekombiniertes Gen eingeführt ist, das das Polynukleotid gemäß einem der Ansprüche 4 bis 11 innerhalb des Chromosoms enthält und das ein neues Collektin ausprägen kann.

## Revendications

1. Nouvelle collectine humaine constituée par la séquence d'acide aminé de la SEQ ID N°: 2, les résidus 206-547.

2. Nouvelle collectine humaine constituée par la séquence d'acide aminé de la SEQ ID N°: 2, les résidus 229-547.

3. Nouvelle collectine selon la revendication 2, dans laquelle ladite nouvelle collectine est, en outre, constituée par une séquence d'acide aminé en amont du premier résidu méthionine (SEQ ID N°: 2, le résidu 229) choisi parmi la SEQ ID N°: 2, les résidus 226-228, 211-228, 102-228, 91-228, 9-228 et 1-228.

4. Polynucléotide isolé constitué par une séquence nucléotidique qui code pour une protéine ayant la séquence d'acide aminé selon l'une quelconque des revendications 1 à 3.

5. Polynucléotide isolé constitué par une séquence nucléotidique ayant la séquence de la SEQ ID N°: 1, les bases 670-1695.

6. Polynucléotide isolé constitué par une séquence nucléotidique ayant la séquence de la SEQ ID N°: 1, les bases 739-1695.

7. Polynucléotide selon la revendication 6, constitué, en outre, par une séquence nucléotidique en 5' en amont de ladite séquence nucléotidique qui est choisie parmi la SEQ ID N° : 1, les bases 730-738, 685-738, 358-738, 325-738, 79-738, 55-738 et 1-738.

8. Polynucléotide selon l'une quelconque des revendications 5 à 7, qui est, en outre, constitué par la séquence nucléotidique de la SEQ ID N°: 1, les bases 1696-2024 en aval de ladite séquence nucléotidique.

9. Polynucléotide isolé codant pour une nouvelle collectine, qui s'hybride avec une sonde qui est un produit d'amplification par une PCR réalisée en utilisant les amorces ayant les séquences nucléotidiques de la SEQ ID N°: 4 et de la SEQ ID N°: 5, sous les conditions d'hybridation suivantes : une préhybridation dans une solution de 5 x SSC, 1% d'agent de blocage, 0,1% de N-lauroyl sarcosine et 0,02% de SDS, à 68°C pendant une heure ; une hybridation dans une solution de 5 x SSC, 1% d'agent de blocage, 0,1% de N-lauroyl sarcosine et 0,02% de SDS contenant les sondes d'ADNc (10 ng/ml), à 55°C pendant 16 heures ; un lavage dans une solution de 2 x SSC/0,1% de SDS pendant 5 minutes, 2 fois ; et un lavage dans une solution de 0,5 x SSC/0,1% de SDS à 55°C pendant 15 minutes, 2 fois.

10. Polynucléotide qui s'hybride avec l'un quelconque des polynucléotides selon l'une quelconque des revendications 4 à 9, sous les conditions d'hybridation suivantes : une préhybridation dans une solution de 5 x SSC, 1% d'agent de blocage, 0,1% de N-lauroyl sarcosine et 0,02% de SDS, à 68°C pendant une heure ; une hybridation dans une solution de 5 x SSC, 1% d'agent de blocage, 0,1% de N-lauroyl sarcosine et 0,02% de SDS contenant les sondes d'ADNc (10 ng/ml), à 55°C pendant 16 heures ; un lavage dans une solution de 2 x SSC/0,1% de SDS pendant 5 minutes, 2 fois ; et un lavage dans une solution de 0,5 x SSC/0,1% de SDS à 55°C pendant 15 minutes, 2 fois, où la protéine codée par ledit polynucléotide est une nouvelle collectine qui comprend : (1) un domaine de reconnaissance d'hydrate de carbone Ca²⁺-dépendant (CRD) et (2) une région de type collagène.

11. Polynucléotide selon l'une quelconque des revendications 4 à 10, dans lesquelles ledit polynucléotide est de l'ADNc.

12. Nouvelle collectine constituée par la séquence d'acide aminé codée par le polynucléotide selon l'une quelconque des revendications 5 à 11.

13. Vecteur qui comprend le polynucléotide selon l'une quelconque des revendications 4 à 11, de manière à permettre l'expression de la nouvelle collectine.

14. Cellule hôte comprenant le vecteur selon la revendication 13, de manière à permettre l'expression de la nouvelle collectine.

15. Sonde pour le criblage d'une espèce moléculaire apparentée à la nouvelle collectine, constituée par le polynucléotide selon l'une quelconque des revendications 4 à 11.

16. Animal transgénique non-humain qui est transfecté, de manière stable, avec un gène recombinant comprenant le polynucléotide selon l'une quelconque des revendications 4 à 11 dans le chromosome et qui peut exprimer une nouvelle collectine.


### SEQUENCE LISTING

<110> FUSO PHARMACEUTICAL INDUSTRIES, LTD.
<120> Novel Collectin
<130> 99P147WO
<150> JP 10-237611
   <151> 1998-08-24
<160> 29
<210> 1
   <211> 2024
   <212> DNA
   <213> Homo Sapiens
<220>
   <221> CDS
   <222> (670).. (1695)
<400> 1
   gtcacgaatc tgcagcaaga taccagcgtg ctccagggca atctgcagaa ccaaatgtat 60
   tctcataatg tggtcatcat gaacctcaac aacctgaacc tgacccaggt gcagcagagg 120
   aacctcatca cgaatctgca gcggtctgtg gatgacacaa gccaggctat ccagcgaatc 180
   aagaacgact ttcaaaatct gcagcaggtt tttcttcaag ccaagaagga cacggattgg 240
   ctgaaggaga aagtgcagag cttgcagacg ctggctgcca acaactctgc gttggccaaa 300
   gccaacaacg acaccctgga ggatatgaac agccagctca actcattcac aggtcagatg 360
   gagaacatca ccactatctc tcaagccaac gagcagaacc tgaaagacct gcaggactta   420
   cacaaagatg cagagaatag aacagccatc aagttcaacc aactggagga acgcttccag   480
   ctctttgaga cggatattgt gaacatcatt agcaatatca gttacacagc ccaccacctg   540
   cggacgctga ccagcaatct aaatgaagtc aggaccactt gcacagatac ccttaccaaa   600
   cacacagatg atctgacctc cttgaataat accctggcca acatccgttt ggattctgtt   660
   tctctcagg atg caa caa gat ttg atg agg tcg agg tta gac act gaa gta   711
             Met Gln Gln Asp Leu Met Arg Ser Arg Leu Asp Thr Glu Val
             1               5                   10
   gcc aac tta tca gtg att atg gaa gaa atg aag cta gta gac tcc aag     759
   Ala Asn Leu Ser Val Ile Met Glu Glu Met Lys Leu Val Asp Ser Lys
   15                  20                  25                  30
   cat ggt cag ctc atc aag aat ttt aca ata cta caa ggt cca ccg ggc     807
   His Gly Gln Leu Ile Lys Asn Phe Thr Ile Leu Gln Gly Pro Pro Gly
                   35                  40                  45
   ccc agg ggt cca aga ggt gac aga gga tcc cag gga ccc cct ggc cca     855
   Pro Arg Gly Pro Arg Gly Asp Arg Gly Ser Gln Gly Pro Pro Gly Pro
               50                  55                  60
   act ggc aac aag gga cag aaa gga gag aag ggg gag cct gga cca cct     903
   Thr Gly Asn Lys Gly Gln Lys Gly Glu Lys Gly Glu Pro Gly Pro Pro
           65                  70                  75
   ggc cct gcg ggt gag aga ggc cca att gga cca gct ggt ccc ccc gga     951
   Gly Pro Ala Gly Glu Arg Gly Pro Ile Gly Pro Ala Gly Pro Pro Gly
       80                  85                  90
   gag cgt ggc ggc aaa gga tct aaa ggc tcc cag ggc ccc aaa ggc tcc     999
   Glu Arg Gly Gly Lys Gly Ser Lys Gly Ser Gln Gly Pro Lys Gly Ser
   95                  100                 105                 110
   cgt ggt tcc cct ggg aag ccc ggc cct cag ggc ccc agt ggg gac cca     1047
   Arg Gly Ser Pro Gly Lys Pro Gly Pro Gln Gly Pro Ser Gly Asp Pro
                   115                 120                 125
   ggc ccc ccg ggc cca cca ggc aaa gag gga ctc ccc ggc cct cag ggc 1095
   Gly Pro Pro Gly Pro Pro Gly Lys Glu Gly Leu Pro Gly Pro Gln Gly
   130 135 140
   cct cct ggc ttc cag gga ctt cag ggc acc gtt ggg gag cct ggg gtg 1143
   Pro Pro Gly Phe Gln Gly Leu Gln Gly Thr Val Gly Glu Pro Gly Val
   145 150 155
   cct gga cct cgg gga ctg cca ggc ttg cct ggg gta cca ggc atg cca 1191
   Pro Gly Pro Arg Gly Leu Pro Gly Leu Pro Gly Val Pro Gly Met Pro
   160 165 170
   ggc ccc aag ggc ccc ccc ggc cct cct ggc cca tca gga gcg gtg gtg 1239
   Gly Pro Lys Gly Pro Pro Gly Pro Pro Gly Pro Ser Gly Ala Val Val
   175 180 185 190
   ccc ctg gcc ctg cag aat gag cca acc ccg gca ccg gag gac aat ggc 1287
   Pro Leu Ala Leu Gln Asn Glu Pro Thr Pro Ala Pro Glu Asp Asn Gly
   195 200 205
   tgc ccg cct cac tgg aag aac ttc aca gac aaa tgc tac tat ttt tca 1335
   Cys Pro Pro His Trp Lys Asn Phe Thr Asp Lys Cys Tyr Tyr Phe Ser
   210 215 220
   gtt gag aaa gaa att ttt gag gat gca aag ctt ttc tgt gaa gac aag 1383
   Val Glu Lys Glu Ile Phe Glu Asp Ala Lys Leu Phe Cys Glu Asp Lys
   225 230 235
   tct tca cat ctt gtt ttc ata aac act aga gag gaa cag caa tgg ata 1431
   Ser Ser His Leu Val Phe Ile Asn Thr Arg Glu Glu Gln Gln Trp Ile
   240 245 250
   aaa aaa cag atg gta ggg aga gag agc cac tgg atc ggc ctc aca gac 1479
   Lys Lys Gln Met Val Gly Arg Glu Ser His Trp Ile Gly Leu Thr Asp
   255 260 265 270
   tca gag cgt gaa aat gaa tgg aag tgg ctg gat ggg aca tct cca gac 1527
   Ser Glu Arg Glu Asn Glu Trp Lys Trp Leu Asp Gly Thr Ser Pro Asp
   275 280 285
   tac aaa aat tgg aaa gct gga cag ccg gat aac tgg ggt cat ggc cat 1575
   Tyr Lys Asn Trp Lys Ala Gly Gln Pro Asp Asn Trp Gly His Gly His
   290 295 300
   ggg cca gga gaa gac tgt gct ggg ttg att tat gct ggg cag tgg aac 1623
   Gly Pro Gly Glu Asp Cys Ala Gly Leu Ile Tyr Ala Gly Gln Trp Asn
   305 310 315
   gat ttc caa tgt gaa gac gtc aat aac ttc att tgc gaa aaa gac agg 1671
   Asp Phe Gln Cys Glu Asp Val Asn Asn Phe Ile Cys Glu Lys Asp Arg
   320 325 330
   gag aca gta ctg tca tct gca tta taacggactg tgatgggatc acatgagcaa 1725
   Glu Thr Val Leu Ser Ser Ala Leu
   335 340
   attttcagct ctcaaaggca aaggacactc ctttctaatt gcatcacctt ctcatcagat 1785
   tgaaaaaaaa aaaagcactg aaaaccaatt actgaaaaaa aattgacagc tagtgttttt 1845
   taccatccgt cattacccaa agacttggga actaaaatgt tccccagggt gatatgctga 1905
   ttttcattgt gcacatggac tgaatcacat agattctcct ccgtcagtaa ccgtgcgatt 1965
   atacaaatta tgtcttccaa agtatggaac actccaatca gaaaaaggtt atcatcccg 2024
<210> 2
   <211> 547
   <212> PRT
   <213> Homo Sapiens
<220>
   <223> Deduced Amino Acid Sequence of Novel Collectin from Nucleotide Sequence.
<400> 2
   Met Tyr Ser His Asn Val Val Ile Met Asn Leu Asn Asn Leu Asn Leu
   1 5 10 15
   Thr Gln Val Gln Gln Arg Asn Leu Ile Thr Asn Leu Gln Arg Ser Val
   20 25 30
   Asp Asp Thr Ser Gln Ala Ile Gln Arg Ile Lys Asn Asp Phe Gln Asn
   35 40 45
   Leu Gln Gln Val Phe Leu Gln Ala Lys Lys Asp Thr Asp Trp Leu Lys
   50 55 60
   Glu Lys Val Gln Ser Leu Gln Thr Leu Ala Ala Asn Asn Ser Ala Leu
   65 70 75 80
   Ala Lys Ala Asn Asn Asp Thr Leu Glu Asp Met Asn Ser Gln Leu Asn
   85 90 95
   Ser Phe Thr Gly Gln Met Glu Asn Ile Thr Thr Ile Ser Gln Ala Asn
   100 105 110
   Glu Gln Asn Leu Lys Asp Leu Gln Asp Leu His Lys Asp Ala Glu Asn
   115 120 125
   Arg Thr Ala Ile Lys Phe Asn Gln Leu Glu Glu Arg Phe Gln Leu Phe
   130 135 140
   Glu Thr Asp Ile Val Asn Ile Ile Ser Asn Ile Ser Tyr Thr Ala His
   145 150 155 160
   His Leu Arg Thr Leu Thr Ser Asn Leu Asn Glu Val Arg Thr Thr Cys
   165 170 175
   Thr Asp Thr Leu Thr Lys His Thr Asp Asp Leu Thr Ser Leu Asn Asn
   180 185 190
   Thr Leu Ala Asn Ile Arg Leu Asp Ser Val Ser Leu Arg Met Gln Gln
   195 200 205
   Asp Leu Met Arg Ser Arg Leu Asp Thr Glu Val Ala Asn Leu Ser Val
   210 215 220
   Ile Met Glu Glu Met Lys Leu Val Asp Ser Lys His Gly Gln Leu Ile
   225 230 235 240
   Lys Asn Phe Thr Ile Leu Gln Gly Pro Pro Gly Pro Arg Gly Pro Arg
   245 250 255
   Gly Asp Arg Gly Ser Gln Gly Pro Pro Gly Pro Thr Gly Asn Lys Gly
   260 265 270
   Gln Lys Gly Glu Lys Gly Glu Pro Gly Pro Pro Gly Pro Ala Gly Glu
   275 280 285
   Arg Gly Pro Ile Gly Pro Ala Gly Pro Pro Gly Glu Arg Gly Gly Lys
   290 295 300
   Gly Ser Lys Gly Ser Gln Gly Pro Lys Gly Ser Arg Gly Ser Pro Gly
   305 310 315 320
   Lys Pro Gly Pro Gln Gly Pro Ser Gly Asp Pro Gly Pro Pro Gly Pro
   325 330 335
   Pro Gly Lys Glu Gly Leu Pro Gly Pro Gln Gly Pro Pro Gly Phe Gln
   340 345 350
   Gly Leu Gln Gly Thr Val Gly Glu Pro Gly Val Pro Gly Pro Arg Gly
   355 360 365
   Leu Pro Gly Leu Pro Gly Val Pro Gly Met Pro Gly Pro Lys Gly Pro
   370 375 380
   Pro Gly Pro Pro Gly Pro Ser Gly Ala Val Val Pro Leu Ala Leu Gln
   385 390 395 400
   Asn Glu Pro Thr Pro Ala Pro Glu Asp Asn Gly Cys Pro Pro His Trp
   405 410 415
   Lys Asn Phe Thr Asp Lys Cys Tyr Tyr Phe Ser Val Glu Lys Glu Ile
   420 425 430
   Phe Glu Asp Ala Lys Leu Phe Cys Glu Asp Lys Ser Ser His Leu Val
   435 440 445
   Phe Ile Asn Thr Arg Glu Glu Gln Gln Trp Ile Lys Lys Gln Met Val
   450 455 460
   Gly Arg Glu Ser His Trp Ile Gly Leu Thr Asp Ser Glu Arg Glu Asn
   465 470 475 480
   Glu Trp Lys Trp Leu Asp Gly Thr Ser Pro Asp Tyr Lys Asn Trp Lys
   485 490 495
   Ala Gly Gln Pro Asp Asn Trp Gly His Gly His Gly Pro Gly Glu Asp
   500 505 510
   Cys Ala Gly Leu Ile Tyr Ala Gly Gln Trp Asn Asp Phe Gln Cys Glu
   515 520 525
   Asp Val Asn Asn Phe Ile Cys Glu Lys Asp Arg Glu Thr Val Leu Ser
   530 535 540
   Ser Ala Leu
   545
<210> 3
   <211> 27
   <212> PRT
   <213> Artificial Sequence
<220>
   <223> Modified Consensus Sequence of collectins Hybridizable with Novel Collectin.
<400> 3
   Glu Lys Cys Val Glu Met Tyr Thr Asp Gly Lys Trp Asn Asp Arg Asn
   1 5 10 15
   Cys Leu Gln Ser Arg Leu Ala Ile Cys Glu Phe
   20 25
<210> 4
   <211> 21
   <212> DNA
   <213> Artificial Sequence
<220>
   <223> Sequence of a Reverse Primer for Screening a Novel Collectin.
<400> 4
   caatctgatg agaaggtgat g 21
<210> 5
   <211> 21
   <212> DNA
   <213> Artificial Sequence
<220>
   <223> Sequence of a Forward Primer for Screening a Novel Collectin.
<400> 5
   acgaggggct ggatgggaca t 21
<210> 6
   <211> 27
   <212> PRT
   <213> Artificial Sequence
<220>
   <223> Consensus sequence of three collectins which were reported heretofore.
<400> 6
   Glu Asp Cys Val Leu Leu Leu Lys Asn Gly Gln Trp Asn Asp Val Pro
   1 5 10 15
   Cys Ser Thr Ser His Leu Ala Val Cys Glu Phe
   20 25
<210> 7
   <211> 24
   <212> DNA
   <213> Artificial Sequence
<220>
   <223> M13 Universal Primer Sequence for Sequencing.
<400> 7
   cgacgttgta aaacgacggc cagt 24
<210> 8
   <211> 17
   <212> DNA
   <213> Artificial Sequence
<220>
   <223> M13 Reverse Primer Sequence for Sequencing.
<400> 8
   caggaaaca gctatgac 17
<210> 9
   <211> 24
   <212> DNA
   <213> Artificial Sequence
<220>
   <223> Sequence of a λgt11 Reverse Primer for Sequencing.
<400> 9
   ttgacaccag accaactggt aatg 24
<210> 10
   <211> 24
   <212> DNA
   <213> Artificial Sequence
<220>
   <223> Sequence of a λgt11 Forward Primer for Sequencing.
<400> 10
   ggtggcgacg actcctggag cccg 24
<210> 11
   <211> 21
   <212> DNA
   <213> Artificial Sequence
<220>
   <223> Sequence of a Primer for Screening a Novel Collectin.
<400> 11
   cgtgaaaatg aatggaagtg g 21
<210> 12
   <211> 21
   <212> DNA
   <213> Artificial Sequence
<220>
   <223> Sequence of a Primer for Screening a Novel Collectin.
<400> 12
   ttttatccat tgctgttcct c 21
<210> 13
   <211> 21
   <212> DNA
   <213> Artificial Sequence
<220>
   <223> Sequence of a Primer for Sequencing a Novel Collectin.
<400> 13
   ctggcagtcc ccgaggtcca g 21
<210> 14
   <211> 21
   <212> DNA
   <213> Artificial Sequence
<220>
   <223> Sequence of a Primer for Sequencing a Novel Collectin.
<400> 14
   gctggtcccc ccggagagcg t 21
<210> 15
   <211> 21
   <212> DNA
   <213> Artificial Sequence
<220>
   <223> Sequence of a 1RC2 Primer for Cap Site Sequencing.
<400> 15
   caaggtacgc cacagcgtat g 21
<210> 16
   <211> 20
   <212> DNA
   <213> Artificial Sequence
<220>
   <223> Sequence of a Synthetic TGP1 Primer for Cap Site Sequencing.
<400> 16
   tcttcagttt ccctaatccc 20
<210> 17
   <211> 21
   <212> DNA
   <213> Artificial Sequence
<220>
   <223> Sequence of a 2RC2 Primer for Cap Site Sequencing.
<400> 17
   gtacgccaca gcgtatgatg c 21
<210> 18
   <211> 21
   <212> DNA
   <213> Artificial Sequence
<220>
   <223> Sequence of a Synthetic TGP2 Primer for Cap Site Sequencing.
<400> 18
   cattcttgac aaacttcata g 21
<210> 19
   <211> 22
   <212> DNA
   <213> Artificial Sequence
<220>
   <223> Sequence of a Primer for Screening a Novel Collectin.
<400> 19
   gaagacaagt cttcaactct tg 22
<210> 20
   <211> 22
   <212> DNA
   <213> Artificial Sequence
<220>
   <223> Sequence of a Primer for Screening a Novel Collectin.
<400> 20
   ctctgagtct gtgaggccga tc 22
<210> 21
   <211> 111
   <212> DNA
   <213> Artificial Sequence
<220>
   <223> Sequence of a Probe for Screening a Novel Collectin.
<400> 21
   gaagacaagt cttcacatct tgttttcata aacactagag aggaacagca atggataaaa 60
   aaacagatgg tagggagaga gagccactgg atcggcctca cagactcaga g 111
<210> 22
   <211> 22
   <212> DNA
   <213> Artificial Sequence
<220>
   <223> Sequence of a Forward Primer for Screening a Novel Collectin.
<400> 22
   gtgcccctgg ccctgcagaa tg 22
<210> 23
   <211> 26
   <212> DNA
   <213> Artificial Sequence
<220>
   <223> Sequence of a Reverse Primer for Screening a Novel Collectin.
<400> 23
   gcatatcacc ctggggaaca ttttag 26
<210> 24
   <211> 21
   <212> DNA
   <213> Artificial Sequence
<220>
   <223> Sequence of a Sense Primer for Screening β-Actin.
<400> 24
   caagagatgg ccacggctgc t 21
<210> 25
   <211> 21
   <212> DNA
   <213> Artificial Sequence
<220>
   <223> Sequence of an Antisense Primer for Screening β-Actin.
<400> 25
   tccttctgca tcctgtcggc a 21
<210> 26
   <211> 39
   <212> DNA
   <213> Artificial Sequence
<220>
   <223> Sequence of a Sense Primer for Amplifying the Novel Collectin.
<400> 26
   aaggaaaaaa gcggccgcat gcaacaagat ttgatgagg 39
<210> 27
   <211> 29
   <212> DNA
   <213> Artificial Sequence
<220>
   <223> Sequence of a Reverse Primer for Amplifying the Novel Collectin.
<400> 27
   gctctagatt ataatgcaga tgacagtac 29
<210> 28
   <211> 21
   <212> DNA
   <213> Artificial Sequence
<220>
   <223> Sequence of a Sense Primer for Amplifying the Nockout Gene.
<400> 28
   atgcaacaag atttgatgag g 21
<210> 29
   <211> 20
   <212> DNA
   <213> Artificial Sequence
<220>
   <223> Sequence of a Sense Primer for Amplifying the Nockout Gene.
<400> 29
   cctacccggt agaattgacc 20

### SEQUENCE LISTING

<110> FUSO PHARMACEUTICAL INDUSTRIES, LTD.
<120> Novel Collectin
<130> 99P147WO
<150> JP 10-237611
   <151> 1998-08-24
<160> 29
<210> 1
   <211> 2024
   <212> DNA
   <213> Homo Sapiens
<220>
   <221> CDS
   <222> (670).. (1695)
<400> 1
   gtcacgaatc tgcagcaaga taccagcgtg ctccagggca atctgcagaa ccaaatgtat     60
   tctcataatg tggtcatcat gaacctcaac aacctgaacc tgacccaggt gcagcagagg    120
   aacctcatca cgaatctgca gcggtctgtg gatgacacaa gccaggctat ccagcgaatc    180
   aagaacgact ttcaaaatct gcagcaggtt tttcttcaag ccaagaagga cacggattgg    240
   ctgaaggaga aagtgcagag cttgcagacg ctggctgcca acaactctgc gttggccaaa    300
   gccaacaacg acaccctgga ggatatgaac agccagctca actcattcac aggtcagatg    360
   gagaacatca ccactatctc tcaagccaac gagcagaacc tgaaagacct gcaggactta   420
   cacaaagatg cagagaatag aacagccatc aagttcaacc aactggagga acgcttccag   480
   ctctttgaga cggatattgt gaacatcatt agcaatatca gttacacagc ccaccacctg   540
   cggacgctga ccagcaatct aaatgaagtc aggaccactt gcacagatac ccttaccaaa   600
   cacacagatg atctgacctc cttgaataat accctggcca acatccgttt ggattctgtt   660
   tctctcagg atg caa caa gat ttg atg agg tcg agg tta gac act gaa gta   711
             Met Gln Gln Asp Leu Met Arg Ser Arg Leu Asp Thr Glu Val
             1               5                   10
   gcc aac tta tca gtg att atg gaa gaa atg aag cta gta gac tcc aag     759
   Ala Asn Leu Ser Val Ile Met Glu Glu Met Lys Leu Val Asp Ser Lys
   15                  20                  25                  30
   cat ggt cag ctc atc aag aat ttt aca ata cta caa ggt cca ccg ggc     807
   His Gly Gln Leu Ile Lys Asn Phe Thr Ile Leu Gln Gly Pro Pro Gly
                   35                  40                  45
   ccc agg ggt cca aga ggt gac aga gga tcc cag gga ccc cct ggc cca     855
   Pro Arg Gly Pro Arg Gly Asp Arg Gly Ser Gln Gly Pro Pro Gly Pro
               50                  55                  60
   act ggc aac aag gga cag aaa gga gag aag ggg gag cct gga cca cct     903
   Thr Gly Asn Lys Gly Gln Lys Gly Glu Lys Gly Glu Pro Gly Pro Pro
           65                  70                  75
   ggc cct gcg ggt gag aga ggc cca att gga cca gct ggt ccc ccc gga     951
   Gly Pro Ala Gly Glu Arg Gly Pro Ile Gly Pro Ala Gly Pro Pro Gly
       80                  85                  90
   gag cgt ggc ggc aaa gga tct aaa ggc tcc cag ggc ccc aaa ggc tcc     999
   Glu Arg Gly Gly Lys Gly Ser Lys Gly Ser Gln Gly Pro Lys Gly Ser
   95                  100                 105                 110
   cgt ggt tcc cct ggg aag ccc ggc cct cag ggc ccc agt ggg gac cca    1047
   Arg Gly Ser Pro Gly Lys Pro Gly Pro Gln Gly Pro Ser Gly Asp Pro
                   115                 120                 125
   ggc ccc ccg ggc cca cca ggc aaa gag gga ctc ccc ggc cct cag ggc 1095
   Gly Pro Pro Gly Pro Pro Gly Lys Glu Gly Leu Pro Gly Pro Gln Gly
   130 135 140
   cct cct ggc ttc cag gga ctt cag ggc acc gtt ggg gag cct ggg gtg 1143
   Pro Pro Gly Phe Gln Gly Leu Gln Gly Thr Val Gly Glu Pro Gly Val
   145 150 155
   cct gga cct cgg gga ctg cca ggc ttg cct ggg gta cca ggc atg cca 1191
   Pro Gly Pro Arg Gly Leu Pro Gly Leu Pro Gly Val Pro Gly Met Pro
   160 165 170
   ggc ccc aag ggc ccc ccc ggc cct cct ggc cca tca gga gcg gtg gtg 1239
   Gly Pro Lys Gly Pro Pro Gly Pro Pro Gly Pro Ser Gly Ala Val Val
   175 180 185 190
   ccc ctg gcc ctg cag aat gag cca acc ccg gca ccg gag gac aat ggc 1287
   Pro Leu Ala Leu Gln Asn Glu Pro Thr Pro Ala Pro Glu Asp Asn Gly
   195 200 205
   tgc ccg cct cac tgg aag aac ttc aca gac aaa tgc tac tat ttt tca 1335
   Cys Pro Pro His Trp Lys Asn Phe Thr Asp Lys Cys Tyr Tyr Phe Ser
   210 215 220
   gtt gag aaa gaa att ttt gag gat gca aag ctt ttc tgt gaa gac aag 1383
   Val Glu Lys Glu Ile Phe Glu Asp Ala Lys Leu Phe Cys Glu Asp Lys
   225 230 235
   tct tca cat ctt gtt ttc ata aac act aga gag gaa cag caa tgg ata 1431
   Ser Ser His Leu Val Phe Ile Asn Thr Arg Glu Glu Gln Gln Trp Ile
   240 245 250
   aaa aaa cag atg gta ggg aga gag agc cac tgg atc ggc ctc aca gac 1479
   Lys Lys Gln Met Val Gly Arg Glu Ser His Trp Ile Gly Leu Thr Asp
   255 260 265 270
   tca gag cgt gaa aat gaa tgg aag tgg ctg gat ggg aca tct cca gac 1527
   Ser Glu Arg Glu Asn Glu Trp Lys Trp Leu Asp Gly Thr Ser Pro Asp
   275 280 285
   tac aaa aat tgg aaa gct gga cag ccg gat aac tgg ggt cat ggc cat 1575
   Tyr Lys Asn Trp Lys Ala Gly Gln Pro Asp Asn Trp Gly His Gly His
   290 295 300
   ggg cca gga gaa gac tgt gct ggg ttg att tat gct ggg cag tgg aac 1623
   Gly Pro Gly Glu Asp Cys Ala Gly Leu Ile Tyr Ala Gly Gln Trp Asn
   305 310 315
   gat ttc caa tgt gaa gac gtc aat aac ttc att tgc gaa aaa gac agg 1671
   Asp Phe Gln Cys Glu Asp Val Asn Asn Phe Ile Cys Glu Lys Asp Arg
   320 325 330
   gag aca gta ctg tca tct gca tta taacggactg tgatgggatc acatgagcaa 1725
   Glu Thr Val Leu Ser Ser Ala Leu
   335 340
   attttcagct ctcaaaggca aaggacactc ctttctaatt gcatcacctt ctcatcagat 1785
   tgaaaaaaaa aaaagcactg aaaaccaatt actgaaaaaa aattgacagc tagtgttttt 1845
   taccatccgt cattacccaa agacttggga actaaaatgt tccccagggt gatatgctga 1905
   ttttcattgt gcacatggac tgaatcacat agattctcct ccgtcagtaa ccgtgcgatt 1965
   atacaaatta tgtcttccaa agtatggaac actccaatca gaaaaaggtt atcatcccg 2024
<210> 2
   <211> 547
   <212> PRT
   <213> Homo Sapiens
<220>
   <223> Deduced Amino Acid Sequence of Novel Collectin from Nucleotide Sequence.
<400> 2
   Met Tyr Ser His Asn Val Val Ile Met Asn Leu Asn Asn Leu Asn Leu
   1 5 10 15
   Thr Gln Val Gln Gln Arg Asn Leu Ile Thr Asn Leu Gln Arg Ser Val
   20 25 30
   Asp Asp Thr Ser Gln Ala Ile Gln Arg Ile Lys Asn Asp Phe Gln Asn
   35 40 45
   Leu Gln Gln Val Phe Leu Gln Ala Lys Lys Asp Thr Asp Trp Leu Lys
   50 55 60
   Glu Lys Val Gln Ser Leu Gln Thr Leu Ala Ala Asn Asn Ser Ala Leu
   65 70 75 80
   Ala Lys Ala Asn Asn Asp Thr Leu Glu Asp Met Asn Ser Gln Leu Asn
   85 90 95
   Ser Phe Thr Gly Gln Met Glu Asn Ile Thr Thr Ile Ser Gln Ala Asn
   100 105 110
   Glu Gln Asn Leu Lys Asp Leu Gln Asp Leu His Lys Asp Ala Glu Asn
   115 120 125
   Arg Thr Ala Ile Lys Phe Asn Gln Leu Glu Glu Arg Phe Gln Leu Phe
   130 135 140
   Glu Thr Asp Ile Val Asn Ile Ile Ser Asn Ile Ser Tyr Thr Ala His
   145 150 155 160
   His Leu Arg Thr Leu Thr Ser Asn Leu Asn Glu Val Arg Thr Thr Cys
   165 170 175
   Thr Asp Thr Leu Thr Lys His Thr Asp Asp Leu Thr Ser Leu Asn Asn
   180 185 190
   Thr Leu Ala Asn Ile Arg Leu Asp Ser Val Ser Leu Arg Met Gln Gln
   195 200 205
   Asp Leu Met Arg Ser Arg Leu Asp Thr Glu Val Ala Asn Leu Ser Val
   210 215 220
   Ile Met Glu Glu Met Lys Leu Val Asp Ser Lys His Gly Gln Leu Ile
   225 230 235 240
   Lys Asn Phe Thr Ile Leu Gln Gly Pro Pro Gly Pro Arg Gly Pro Arg
   245 250 255
   Gly Asp Arg Gly Ser Gln Gly Pro Pro Gly Pro Thr Gly Asn Lys Gly
   260 265 270
   Gln Lys Gly Glu Lys Gly Glu Pro Gly Pro Pro Gly Pro Ala Gly Glu
   275 280 285
   Arg Gly Pro Ile Gly Pro Ala Gly Pro Pro Gly Glu Arg Gly Gly Lys
   290 295 300
   Gly Ser Lys Gly Ser Gln Gly Pro Lys Gly Ser Arg Gly Ser Pro Gly
   305 310 315 320
   Lys Pro Gly Pro Gln Gly Pro Ser Gly Asp Pro Gly Pro Pro Gly Pro
   325 330 335
   Pro Gly Lys Glu Gly Leu Pro Gly Pro Gln Gly Pro Pro Gly Phe Gln
   340 345 350
   Gly Leu Gln Gly Thr Val Gly Glu Pro Gly Val Pro Gly Pro Arg Gly
   355 360 365
   Leu Pro Gly Leu Pro Gly Val Pro Gly Met Pro Gly Pro Lys Gly Pro
   370 375 380
   Pro Gly Pro Pro Gly Pro Ser Gly Ala Val Val Pro Leu Ala Leu Gln
   385 390 395 400
   Asn Glu Pro Thr Pro Ala Pro Glu Asp Asn Gly Cys Pro Pro His Trp
   405 410 415
   Lys Asn Phe Thr Asp Lys Cys Tyr Tyr Phe Ser Val Glu Lys Glu Ile
   420 425 430
   Phe Glu Asp Ala Lys Leu Phe Cys Glu Asp Lys Ser Ser His Leu Val
   435 440 445
   Phe Ile Asn Thr Arg Glu Glu Gln Gln Trp Ile Lys Lys Gln Met Val
   450 455 460
   Gly Arg Glu Ser His Trp Ile Gly Leu Thr Asp Ser Glu Arg Glu Asn
   465 470 475 480
   Glu Trp Lys Trp Leu Asp Gly Thr Ser Pro Asp Tyr Lys Asn Trp Lys
   485 490 495
   Ala Gly Gln Pro Asp Asn Trp Gly His Gly His Gly Pro Gly Glu Asp
   500 505 510
   Cys Ala Gly Leu Ile Tyr Ala Gly Gln Trp Asn Asp Phe Gln Cys Glu
   515 520 525
   Asp Val Asn Asn Phe Ile Cys Glu Lys Asp Arg Glu Thr Val Leu Ser
   530 535 540
   Ser Ala Leu
   545
<210> 3
   <211> 27
   <212> PRT
   <213> Artificial Sequence
<220>
   <223> Modified Consensus Sequence of collectins Hybridizable with Novel Collectin.
<400> 3
   Glu Lys Cys Val Glu Met Tyr Thr Asp Gly Lys Trp Asn Asp Arg Asn
   1 5 10 15
   Cys Leu Gln Ser Arg Leu Ala Ile Cys Glu Phe
   20 25
<210> 4
   <211> 21
   <212> DNA
   <213> Artificial Sequence
<220>
   <223> Sequence of a Reverse Primer for Screening a Novel Collectin.
<400> 4
   caatctgatg agaaggtgat g 21
<210> 5
   <211> 21
   <212> DNA
   <213> Artificial Sequence
<220>
   <223> Sequence of a Forward Primer for Screening a Novel Collectin.
<400> 5
   acgaggggct ggatgggaca t 21
<210> 6
   <211> 27
   <212> PRT
   <213> Artificial Sequence
<220>
   <223> Consensus sequence of three collectins which were reported heretofore.
<400> 6
   Glu Asp Cys Val Leu Leu Leu Lys Asn Gly Gln Trp Asn Asp Val Pro
   1 5 10 15
   Cys Ser Thr Ser His Leu Ala Val Cys Glu Phe
   20 25
<210> 7
   <211> 24
   <212> DNA
   <213> Artificial Sequence
<220>
   <223> M13 Universal Primer Sequence for Sequencing.
<400> 7
   cgacgttgta aaacgacggc cagt 24
<210> 8
   <211> 17
   <212> DNA
   <213> Artificial Sequence
<220>
   <223> M13 Reverse Primer Sequence for Sequencing.
<400> 8
   caggaaaca gctatgac 17
<210> 9
   <211> 24
   <212> DNA
   <213> Artificial Sequence
<220>
   <223> Sequence of a λgt11 Reverse Primer for Sequencing.
<400> 9
   ttgacaccag accaactggt aatg 24
<210> 10
   <211> 24
   <212> DNA
   <213> Artificial Sequence
<220>
   <223> Sequence of a λgt11 Forward Primer for Sequencing.
<400> 10
   ggtggcgacg actcctggag cccg 24
<210> 11
   <211> 21
   <212> DNA
   <213> Artificial Sequence
<220>
   <223> Sequence of a Primer for Screening a Novel Collectin.
<400> 11
   cgtgaaaatg aatggaagtg g 21
<210> 12
   <211> 21
   <212> DNA
   <213> Artificial Sequence
<220>
   <223> Sequence of a Primer for Screening a Novel Collectin.
<400> 12
   ttttatccat tgctgttcct c 21
<210> 13
   <211> 21
   <212> DNA
   <213> Artificial Sequence
<220>
   <223> Sequence of a Primer for Sequencing a Novel Collectin.
<400> 13
   ctggcagtcc ccgaggtcca g 21
<210> 14
   <211> 21
   <212> DNA
   <213> Artificial Sequence
<220>
   <223> Sequence of a Primer for Sequencing a Novel Collectin.
<400> 14
   gctggtcccc ccggagagcg t 21
<210> 15
   <211> 21
   <212> DNA
   <213> Artificial Sequence
<220>
   <223> Sequence of a 1RC2 Primer for Cap Site Sequencing.
<400> 15
   caaggtacgc cacagcgtat g 21
<210> 16
   <211> 20
   <212> DNA
   <213> Artificial Sequence
<220>
   <223> Sequence of a Synthetic TGP1 Primer for Cap Site Sequencing.
<400> 16
   tcttcagttt ccctaatccc 20
<210> 17
   <211> 21
   <212> DNA
   <213> Artificial Sequence
<220>
   <223> Sequence of a 2RC2 Primer for Cap Site Sequencing.
<400> 17
   gtacgccaca gcgtatgatg c 21
<210> 18
   <211> 21
   <212> DNA
   <213> Artificial Sequence
<220>
   <223> Sequence of a Synthetic TGP2 Primer for Cap Site Sequencing.
<400> 18
   cattcttgac aaacttcata g 21
<210> 19
   <211> 22
   <212> DNA
   <213> Artificial Sequence
<220>
   <223> Sequence of a Primer for Screening a Novel Collectin.
<400> 19
   gaagacaagt cttcaactct tg 22
<210> 20
   <211> 22
   <212> DNA
   <213> Artificial Sequence
<220>
   <223> Sequence of a Primer for Screening a Novel Collectin.
<400> 20
   ctctgagtct gtgaggccga tc 22
<210> 21
   <211> 111
   <212> DNA
   <213> Artificial Sequence
<220>
   <223> Sequence of a Probe for Screening a Novel Collectin.
<400> 21
   gaagacaagt cttcacatct tgttttcata aacactagag aggaacagca atggataaaa 60
   aaacagatgg tagggagaga gagccactgg atcggcctca cagactcaga g 111
<210> 22
   <211> 22
   <212> DNA
   <213> Artificial Sequence
<220>
   <223> Sequence of a Forward Primer for Screening a Novel Collectin.
<400> 22
   gtgcccctgg ccctgcagaa tg 22
<210> 23
   <211> 26
   <212> DNA
   <213> Artificial Sequence
<220>
   <223> Sequence of a Reverse Primer for Screening a Novel Collectin.
<400> 23
   gcatatcacc ctggggaaca ttttag 26
<210> 24
   <211> 21
   <212> DNA
   <213> Artificial Sequence
<220>
   <223> Sequence of a Sense Primer for Screening β-Actin.
<400> 24
   caagagatgg ccacggctgc t 21
<210> 25
   <211> 21
   <212> DNA
   <213> Artificial Sequence
<220>
   <223> Sequence of an Antisense Primer for Screening β-Actin.
<400> 25
   tccttctgca tcctgtcggc a 21
<210> 26
   <211> 39
   <212> DNA
   <213> Artificial Sequence
<220>
   <223> Sequence of a Sense Primer for Amplifying the Novel Collectin.
<400> 26
   aaggaaaaaa gcggccgcat gcaacaagat ttgatgagg 39
<210> 27
   <211> 29
   <212> DNA
   <213> Artificial Sequence
<220>
   <223> Sequence of a Reverse Primer for Amplifying the Novel Collectin.
<400> 27
   gctctagatt ataatgcaga tgacagtac 29
<210> 28
   <211> 21
   <212> DNA
   <213> Artificial Sequence
<220>
   <223> Sequence of a Sense Primer for Amplifying the Nockout Gene.
<400> 28
   atgcaacaag atttgatgag g 21
<210> 29
   <211> 20
   <212> DNA
   <213> Artificial Sequence
<220>
   <223> Sequence of a Sense Primer for Amplifying the Nockout Gene.
<400> 29
   cctacccggt agaattgacc 20